# EUROPEAN PATENT APPLICATION

(11) **EP 4 678 111 A1**
(43) Date of publication of application: **14.01.2026**
(21) Application number: 25189003.4
(22) Date of filing: 11.07.2025
(51) Int. Cl.: A61B 6/04, A61B 6/00

(54) **X-RAY IMAGING SYSTEM AND IMAGING METHOD THEREOF**

(30) Priority: 11.07.2024 CN 202410927902
(71) Applicant: Shenzhen Mindray Bio-Medical Electronics Co., Ltd., Shenzhen, Guangdong 518057 (CN)
(72) Inventor: ZHANG, Zanchao, Shenzhen, 518057 (CN); LIU, Yanpeng, Shenzhen, 518057 (CN)
(74) Representative: KIPA AB

(57) **Abstract**

Disclosed are an X-ray imaging system and an imaging method thereof, wherein: a bed platform supports an object under examination in a supine posture along a first direction and has first and second sides along the first direction; an X-ray head is capable of being positioned at the first side via a first support assembly, and a detector is capable of being positioned at the second side via a second support assembly, such that the X-ray and detector face a lateral view of the object to obtain a lateral X-ray image; in response to an exposure instruction for a first body part to be stitched, the X-ray head and detector are controlled to cooperatively image the first body part to obtain at least two lateral X-ray images stitched to obtain a radiograph. This enables imaging and stitching of the lateral view of the object laid supine.

## Description

### TECHNICAL FIELD

The present disclosure relates to radiation imaging, and particularly to X-ray imaging systems and imaging methods thereof.

### BACKGROUND OF THE DISCLOSURE

Radiation ray imaging equipment, as a common imaging system in medical digital imaging, is widely applied in the fields of physical examinations and routine medical imaging diagnosis. The radiation ray imaging equipment refers to a device that utilizes radioactive rays (e.g., X-rays) penetrating through an object under examination for imaging. Taking the X-ray imaging system as an example, it has gained extensive clinical applications due to its advantages including fast imaging speed, low radiation dose, high-definition and detailed images, and full-body examination capability, thereby becoming one of the primary auxiliary tools for doctors in disease diagnosis. Typically, the X-ray imaging system comprises an X-ray head and a detector. The X-ray head serves as a component for emitting X-rays, while the detector functions as a component for receiving X-rays penetrating the object under examination. The detector forms images by receiving the X-rays and ultimately converting them into electrical signals.

According to diagnostic requirements, it is necessary to perform anteroposterior or lateral projections of an object under examination (e.g. a patient). An anteroposterior projection (also referred to as imaging an anteroposterior projection of the object under examination) refers to imaging performed along the front-to-back or back-to-front direction of the object under examination, i.e., capturing the coronal plane of the object under examination. A lateral projection (also referred to as imaging a lateral view of the object under examination) refers to imaging performed along the left-to-right or right-to-left direction of the object under examination, i.e., capturing the sagittal plane of the object under examination. Furthermore, depending on whether the object under examination is standing or lying on a bed during imaging, the procedures are classified into standing-position imaging and supine-position imaging.

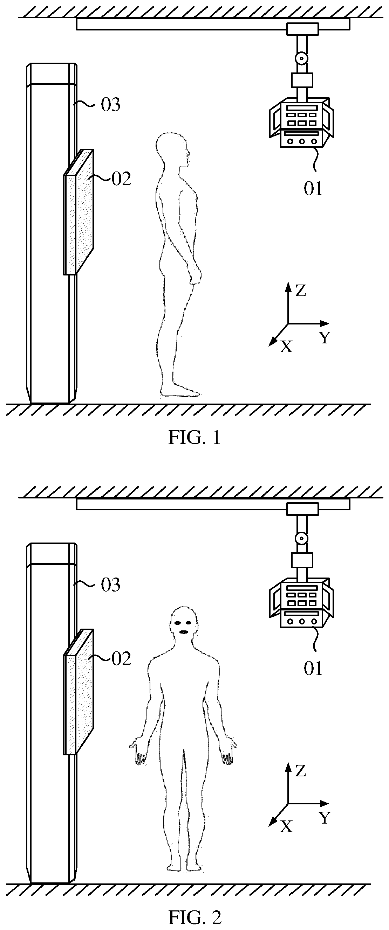

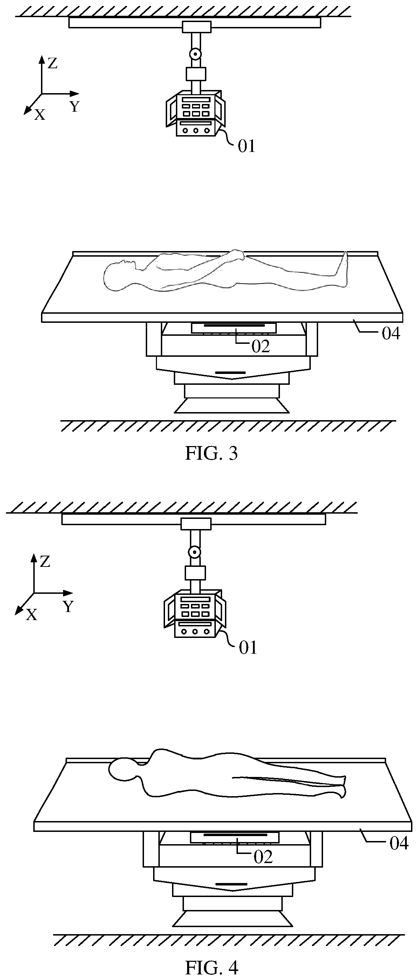

Please refer to FIGS. 1 and 2, which illustrate two examples of standing-position imaging, specifically standing anteroposterior projection and standing lateral projection respectively. FIGS. 3 and 4 show two examples of supine-position imaging, specifically supine anteroposterior projection and supine lateral projection respectively. In the figures, the Z-axis direction represents the vertical orientation while the XY-plane corresponds to the horizontal orientation. In FIG. 1, a detector 02 is disposed on a vertical column 03, the position and orientation of an X-ray head 01 are adjusted so that the X-ray head 01 is faced toward the object under examination along the Y-axis to capture the anterior-posterior view of the object under examination, i.e., imaging the anteroposterior view of the object under examination. In FIG. 2, the position and orientation of the X-ray head 01 are adjusted so that X-ray head 01 is faced toward the object under examination along the Y-axis to capture the lateral view of the object under examination from the right side thereof, i.e., imaging the lateral view of the object under examination. In certain clinical scenarios where the object under examination sustains lower limb injuries or other medical conditions that preclude standing (i.e., inability to maintain an upright posture), the object under examination may be positioned to lie on a bed plate 04 for radiographic imaging. In FIG. 3, the object under examination lies supine on the bed plate 04 with anterior surface facing upward; and the X-ray head 01 is aligned along Z-axis (vertical direction) to capture the anterior-posterior view of the object under examination, i.e., imaging the anteroposterior view of the object under examination. In FIG. 4, the object under examination is in a left lateral recumbent position on bed plate 04, with the anterior surface facing the X-axis (i.e., the leftward direction of the bed); and the X-ray head 01 is aligned along the Z-axis (vertical direction to capture the lateral view of the object under examination, i.e., imaging the lateral view of the object under examination. However, even when in a supine position, due to illness or injury and other reasons, the object under examination still cannot maintain the lateral lying position as shown in FIG. 4 independently, which makes it impossible to capture the lateral view of the object under examination smoothly.

### SUMMARY OF THE DISCLOSURE

Considering the above problem, X-ray imaging systems and imaging methods thereof are provided by the present disclosure, as detailed below.

According to a first aspect of the present disclosure, an X-ray imaging system provided in some embodiments may include an X-ray head device, an X-ray receiving device, and a processor; wherein
the X-ray head device comprises a first support assembly, a first drive mechanism, and an X-ray head; wherein the X-ray head is configured to emit X-rays to an object under examination and is movably disposed on the first support assembly, and the first drive mechanism is configured to drive the X-ray head to move;
the X-ray receiving device comprises a bed platform, a second support assembly, and a detector; wherein the bed platform is configured to support the object under examination who is lying in a supine posture along a first direction of the bed platform, the bed platform has a first side and a second side that are distributed along its first direction, the first side and the second side being opposite sides of the bed platform; the second support assembly is configured to support the detector; the detector is configured to receive X-rays penetrating the object under examination; the X-ray head is capable of being positioned at the first side of the bed platform via the first support assembly, and the the detector is capable of being positioned at the second side of the bed platform via the second support assembly, such that the X-ray head and the detector are faced toward a lateral view of the object under examination in a supine posture to obtain a lateral X-ray image of the object under examination; and
the processor is configured to:
   acquire a first body part to be stitched of the object under examination;
   acquire an exposure parameter set of the first body part to be stitched, wherein the exposure parameter set comprises: a plurality of first travel points, to which the X-ray head is controlled to move, that are distributed at the first side of the bed platform along the first direction, and exposure parameters for the X-ray head at at least two of the first travel points;
   in response to an exposure instruction for the first body part to be stitched, control, based on the exposure parameter set, the first drive mechanism to drive the X-ray head to pass each of the at least two of the first travel points, and when the X-ray head reaches each first travel point, control, based on the exposure parameters for that first travel point, the X-ray head to emit X-rays to the first body part to be stitched of the object under examination in a supine posture;
   control the detector at the second side of the bed platform to receive X-rays penetrating the first body part to be stitched, thereby obtaining at least two lateral X-ray images of the first body part to be stitched; and
   stitch the at least two lateral X-ray images to obtain a radiograph of the first body part to be stitched.

In some embodiments, the X-ray receiving device further comprises a second drive mechanism configured to drive the detector to move; and the processor being configured to control the detector at the second side of the bed platform to receive X-rays penetrating the first body part to be stitched, thereby obtaining at least two lateral X-ray images of the first body part to be stitched, comprises:
the processor being configured to control the second drive mechanism to drive the detector to move to a position corresponding to each first travel point where the X-ray head passes and emits X-rays, and to receive X-rays emitted by the X-ray head at said first travel point and penetrating the first body part to be stitched, thereby obtaining the at least two lateral X-ray images;
or, the processor being configured to control the detector to remain stationary at the second side of the bed platform, and control the detector to receive the X-rays emitted by the X-ray head at each of the at least two of the first travel points where the X-ray head is moved and penetrating the first body part to be stitched, thereby obtaining the at least two lateral X-ray images.

In some embodiments, the X-ray imaging system further comprises:
a first positioning button configured to be triggered by an operator to cause the processor to control the first drive mechanism to drive the X-ray head to move to the first side of the bed platform; and
an image capturing device configured to capture a first image that comprises at least one of: an optical image with two-dimensional spatial information, and a depth image with three-dimensional spatial information; wherein the processor is further configured to perform a first path planning based on the first image to control the first drive mechanism to drive the X-ray head to move to the first side of the bed platform.

In some embodiments, the X-ray receiving device further comprises a second drive mechanism configured to drive the detector to move;
the X-ray imaging system further comprises a second positioning button configured to be triggered by an operator to cause the processor to control the second drive mechanism to drive the detector to move to the second side of the bed platform, wherein the second positioning button and the first positioning button are a same functional button or different functional buttons;
and/or, the processor is further configured to perform a second path planning based on the first image to control the second drive mechanism to drive the detector to move to the second side of the bed platform.

In some embodiments, the X-ray imaging system further comprises an image capturing device configured to capture a second image that comprises at least one of: an optical image with two-dimensional spatial information, and a depth image with three-dimensional spatial information; wherein the determination of the exposure parameter set of the first body part to be stitched by the processor comprises: the processor being configured to acquire a current positional relationship between the first body part to be stitched and the detector based on the second image, and determine the exposure parameter set of the first body part to be stitched according to the current positional relationship;
or, the X-ray imaging system further comprises at least a base station disposed on the X-ray head and at least a tag disposed on the detector, wherein the base station is configured to transmit signals to the tag and receive signals from the tag, the processor is further configured to calculate relative position information between the base station and the tag based on the signals transmitted and received by the base station; and the processor being configured to determine the exposure parameter set of the first body part to be stitched comprises: the processor being configured to acquire a current positional relationship between the first body part to be stitched and the detector based on the relative position information, and determine the exposure parameter set of the first body part to be stitched according to the current positional relationship;
or, the processor being configured to determine the exposure parameter set of the first body part to be stitched comprises: the processor being configured to acquire a predetermined positional relationship between the first body part to be stitched and the detector when the object under examination is in a supine posture, and determine the exposure parameter set of the first body part to be stitched according to the predetermined positional relationship.

In some embodiments, the processor is further configured to acquire a region of interest of the first body part to be stitched; and stitching the at least two lateral X-ray images by the processor to obtain the radiograph of the first body part to be stitched comprises: determining a stitching region between adjacent lateral X-ray images based on the region of interest, such that the region of interest of the first body part to be stitched is located outside the stitching region.

In some embodiments, the second support assembly comprises a mounting bracket configured to detachably mount the detector, wherein the mounting bracket is disposed at the second side of the bed platform and movable along the first direction, so that the detector is capable of being positioned at the second side of the bed platform and driven to move with the mounting bracket.

In some embodiments, the X-ray imaging system further comprises at least a base station disposed on the X-ray head, and at least a tag disposed on the detector; wherein the base station is configured to transmit signals to the tag and receive signals from the tag; and
the processor is further configured to: calculate relative position information between the base station and the tag based on the signals transmitted and received by the base station; and control the first drive mechanism to drive the X-ray head to move according to the relative position information, so that the X-ray head and the detector satisfy a positional matching relationship required before imaging.

In some embodiments, the positional matching relationship required before imaging comprises: the X-ray head and the detector satisfying a predetermined distance requirement and/or a predetermined angular requirement.

In some embodiments, the X-ray head is further capable of being positioned above the bed platform via the first support assembly, and the detector is further capable of being positioned below the bed platform via the second support assembly, such that the X-ray head and the detector are faced toward the anteroposterior view of the object under examination in a supine posture to obtain an anteroposterior X-ray image of the object under examination.

According to a second aspect of the present disclosure, an X-ray imaging system provided in some embodiments may comprise an X-ray head device, a X-ray receiving device, and a processor; wherein
the X-ray head device comprises a first support assembly configured to emit X-rays to an object under examination, and an X-ray head movably disposed on the first support assembly;
the X-ray receiving device comprises a bed platform, a second support assembly, and a detector; wherein the bed platform is configured to support the object under examination who is lying in a supine posture along a first direction of the bed platform, the bed platform has a first side and a second side that are distributed along its first direction, the first side and the second side are opposite sides of the bed platform; the second support assembly is configured to support the detector; the detector is configured to receive X-rays penetrating the object under examination; wherein the X-ray head is capable of being positioned at the first side of the bed platform via the first support assembly, and the detector is capable of being positioned at the second side of the bed platform via the second support assembly, such that the X-ray head and the detector are faced toward a lateral view of the object under examination in a supine posture to obtain a lateral X-ray image of the object under examination;
the processor is configured to:
   acquire a first body part to be stitched of the object under examination;
   in response to an exposure instruction for the first body part to be stitched, control the X-ray head and the detector to cooperatively image the first body part to be stitched of the object under examination in a supine posture, thereby obtaining at least two lateral X-ray images of the first body part to be stitched; and
   stitch the at least two lateral X-ray images to obtain a radiograph of the first body part to be stitched.

In some embodiments, the processor being configured to control the X-ray head and the detector to cooperatively image the first body part to be stitched of the object under examination in a supine posture, thereby obtaining at least two lateral X-ray images of the first body part to be stitched, comprises:
the processor being configured to control a first drive mechanism to drive the X-ray head to move to a plurality of first travel points that are distributed at the first side of the bed platform along the first direction; and when the X-ray head is moved to each of at least two first travel points, control the X-ray head to emit the X-rays to the first body part to be stitched, wherein the X-ray head device further comprises the first drive mechanism;
or, the X-ray head is manually moved by an operator-applied force to a plurality of first travel points that are distributed at the first side of the bed platform along the first direction; and when the X-ray head is moved to each of at least two first travel points, the processor is configured to control the X-ray head to emit X-rays to the first body part to be stitched.

In some embodiments, the processor being configured to control the X-ray head and the detector by the processor to cooperatively image the first body part to be stitched of the object under examination in a supine posture, thereby obtaining at least two lateral X-ray images of the first body part to be stitched further comprises:
the processor being configured to control a second drive mechanism to drive the detector to move to a position corresponding to each first travel point where the X-ray head passes and emits X-rays and to receive the X-rays emitted by the X-ray head at said first travel point and penetrating the first body part to be stitched, thereby obtaining the at least two lateral X-ray images, wherein the X-ray receiving device further comprises the second drive mechanism;
or, the processor being configured to control the detector to remain stationary at the second side of the bed platform, and control the detector to receive the X-rays emitted by the X-ray head at each first travel point where the X-ray head passes and penetrating the first body part to be stitched, thereby obtaining the at least two lateral X-ray images.

In some embodiments, the X-ray imaging system further comprises: a beam-limiting window assembly disposed between the first body part to be stitched and the X-ray head, wherein the beam-limiting window assembly comprises a shielding portion for blocking X-rays and a window defined by the shielding portion for allowing passage of X-rays; and
controlling the X-ray head and the detector by the processor to cooperatively image the first body part to be stitched of the object under examination in a supine posture, thereby obtaining at least two lateral X-ray images of the first body part to be stitched comprises:
the X-ray head remaining stationary at the first side of the bed platform, the window of the beam-limiting window assembly being controlled to sequentially move to multiple first positions distributed along the first direction, the detector being controlled to sequentially move to multiple second positions corresponding to the multiple first positions and receive X-rays penetrating the first body part to be stitched via the window at each of the second positions, thereby obtaining the at least two lateral X-ray images.

According to a third aspect of the present disclosure, an X-ray imaging system provided in some embodiments may include a X-ray head device, an X-ray receiving device, and a processor; wherein
the X-ray head device comprises a first support assembly, and an X-ray head that is configured to emit X-rays to an object under examination and movably disposed on the first support assembly;
the X-ray receiving device comprises a bed platform, a second support assembly, and a detector; wherein the bed platform is configured to support the object under examination who is lying in a supine posture along a first direction of the bed platform, the bed platform has a first side and a second side that are distributed along its first direction, the first side and the second side being opposite sides of the bed platform; the second support assembly is configured to support the detector; the detector is configured to receive X-rays penetrating the object under examination, wherein the X-ray head is capable of being positioned at the first side of the bed platform via the first support assembly, and the detector is capable of being positioned at the second side of the bed platform via the second support assembly, such that the X-ray head and the detector are faced toward a lateral view of the object under examination in a supine posture to obtain a lateral X-ray image of the object under examination; and wherein the X-ray head is further capable of being positioned above the bed platform via the first support assembly, and the detector is further capable of being positioned below the bed platform via the second support assembly, such that the X-ray head and the detector are faced toward the anteroposterior view of the object under examination in a supine posture to obtain an anteroposterior X-ray image of the object under examination;
the X-ray imaging system has a first imaging mode for capturing a first body part to be stitched and a second imaging mode for capturing a second body part to be stitched; the processor is configured to:
   control the X-ray head and the detector to cooperatively image the first body part to be stitched of the object under examination in a supine posture, thereby obtaining at least two lateral X-ray images of the first body part to be stitched; wherein a radiograph of the first body part to be stitched is obtained by stitching the at least two lateral X-ray images by the processor; and
   control the X-ray head and the detector to cooperatively image the second body part to be stitched of the object under examination in a supine posture, thereby obtaining at least two anteroposterior X-ray images of the second body part to be stitched; wherein a radiograph of the second body part to be stitched is obtained by stitching the at least two anteroposterior X-ray images by the processor.

In some embodiments, the processor being configured to control the X-ray head and the detector by the processor to cooperatively image the first body part to be stitched of the object under examination in a supine posture, thereby obtaining at least two lateral X-ray images of the first body part to be stitched, comprises:
the processor being configured to control a first drive mechanism to drive the X-ray head to move to a plurality of first travel points that are distributed at the first side of the bed platform along the first direction; and when the X-ray head is moved to each of at least two first travel points, control the X-ray head to emit the X-rays to the first body part to be stitched; wherein the X-ray head device further comprises the first drive mechanism;
or, the X-ray head is manually moved by an operator-applied force to a plurality of first travel points that are distributed at the first side of the bed platform along the first direction, and when the X-ray head is moved to each of at least two first travel points, the processor is configured to control the X-ray head to emit X-rays to the first body part to be stitched.

In some embodiments, the processor is configured to control the X-ray head and the detector by the processor to cooperatively image the first body part to be stitched of the object under examination in a supine posture, thereby obtaining at least two lateral X-ray images of the first body part to be stitched further comprises:
the processor being configured to control a second drive mechanism to drive the detector to move to a position corresponding to each first travel point where the X-ray head passes and emits X-rays and to receive X-rays emitted by the X-ray head at said first travel point and penetrating the first body part to be stitched, thereby obtaining the at least two lateral X-ray images; wherein the X-ray receiving device further comprises the second drive mechanism;
or, the processor being configured to control the detector to remain stationary at the second side of the bed platform, and control the detector to receive X-rays emitted by the X-ray head at each first travel point where the X-rays passes and penetrating the first body part to be stitched, thereby obtaining the at least two lateral X-ray images.

In some embodiments, the X-ray head device further comprises a first drive mechanism configured to drive the X-ray head to move; the X-ray imaging system further comprises a mode switching interface that is capable of being triggered by an operator to switch imaging modes; wherein, in response to triggering of the mode switching interface, the processor is configured to determine the current imaging mode, wherein
when the current imaging mode is the first imaging mode, the processor is configured to control the first drive mechanism to drive the X-ray head to move to the first side of the bed platform; and
when the current imaging mode is the second imaging mode, the processor is configured to control the first drive mechanism to drive the X-ray head to move to a position over the bed platform.

In some embodiments, the X-ray receiving device further comprises a second drive mechanism configured to drive the detector to move;
when the current imaging mode is the first imaging mode, the processor is further configured to control the second drive mechanism to drive the detector to move to the second side of the bed platform; and
when the current imaging mode is the second imaging mode, the processor is configured to control the second drive mechanism to drive the detector to move to a position below the bed platform.

In some embodiments, the X-ray head device further comprises a first drive mechanism configured to drive the X-ray head to move; the X-ray imaging system further comprises an image capturing device configured to capture a first image that comprises at least one of an optical image with two-dimensional spatial information, and a depth image with three-dimensional spatial information;
when the current imaging mode is the first imaging mode, the processor is configured to perform a first path planning based on the first image to control the first drive mechanism to drive the X-ray head to move to the first side of the bed platform; and
when the current imaging mode is the second imaging mode, the processor is configured to perform a third path planning based on the first image to control the first drive mechanism to drive the X-ray head to move to a position over the bed platform.

In some embodiments, the X-ray receiving device further comprises a second drive mechanism configured to drive the detector to move;
when the current imaging mode is the first imaging mode, the processor is further configured to perform a second path planning based on the first image to control the second drive mechanism to drive the detector to move to the second side of the bed platform; and
when the current imaging mode is the second imaging mode, the processor is further configured to perform a fourth path planning based on the first image to control the second drive mechanism to drive the detector to move to a position below the bed platform.

According to a fourth aspect of the present disclosure, an imaging method for an X-ray imaging system is provided in some embodiments, wherein the X-ray imaging system comprises an X-ray head, a detector, and a bed platform that is configured to support an object under examination who is lying in a supine posture along a first direction of the bed platform, the bed platform further has a first side and a second side that are distributed along its first direction, the first side and the second side are opposite sides of the bed platform; the imaging method comprising:
acquiring a first body part to be stitched of the object under examination;
determining an exposure parameter set of the first body part to be stitched, wherein the exposure parameter set comprises: a plurality of first travel points, to which the X-ray head is controlled to move, that are distributed at the first side of the bed platform along the first direction, and exposure parameters for the X-ray head at at least two of the first travel points;
in response to an exposure instruction for the first body part to be stitched, controlling, based on the exposure parameter set, the X-ray head to pass each of the at least two of the first travel points, and when the X-ray head is moved to each first travel point, controlling, based on the exposure parameters for said first travel point, the X-ray head to emit X-rays to the first body part to be stitched of the object under examination in a supine posture;
controlling the detector at the second side of the bed platform to receive X-rays penetrating the first body part to be stitched, thereby obtaining at least two lateral X-ray images of the first body part to be stitched; and
stitching the at least two lateral X-ray images to obtain a radiograph of the first body part to be stitched.

In some embodiments, said controlling the detector at the second side of the bed platform to receive X-rays penetrating the first body part to be stitched, thereby obtaining at least two lateral X-ray images of the first body part to be stitched comprises:
controlling the detector to move to a position corresponding to each first travel point where the X-ray head passes and emits X-rays and receive X-rays emitted by the X-ray head at said first travel point and penetrating the first body part to be stitched, thereby obtaining the at least two lateral X-ray images;
or, controlling the detector to remain stationary at the second side of the bed platform, and controlling the detector to receive X-rays emitted by the X-ray head at each of the at least two of the first travel points where the X-ray head is moved and penetrating the first body part to be stitched, thereby obtaining the at least two lateral X-ray images.

In some embodiments, the imaging method further comprises:
before imaging: controlling the X-ray head to move to the first side of the bed platform in response to triggering of a first positioning button; and/or controlling the detector to move to the second side of the bed platform in response to triggering of a second positioning button; wherein the second positioning button and the first positioning button are a same functional button or different functional buttons;
or, before imaging: obtaining a first image that comprises at least one of an optical image with two-dimensional spatial information and a depth image with three-dimensional spatial information; performing a first path planning based on the first image to control the X-ray head to move to the first side of the bed platform, and/or, performing a second path planning based on the first image to control the detector to move to the second side of the bed platform.

In some embodiments, the imaging method further comprises: acquiring a region of interest of the first body part to be stitched;
wherein said stitching the at least two lateral X-ray images to obtain a radiograph of the first body part to be stitched comprises: determining a stitching region between adjacent lateral X-ray images based on the region of interest, such that the region of interest of the first body part to be stitched is located outside the stitching region.

According to a fifth aspect of the present disclosure, an imaging method for an X-ray imaging system is provided in some embodiments, wherein the the X-ray imaging system comprises an X-ray head, a detector, and a bed platform that is configured to support an object under examination who is lying in a supine posture along a first direction of the bed platform, the bed platform further has a first side and a second side that are distributed along its first direction, the first side and the second side are opposite sides of the bed platform; the imaging method comprising:
acquiring a first body part to be stitched of the object under examination;
in response to an exposure instruction for the first body part to be stitched, controlling the X-ray head and the detector to cooperatively image the first body part to be stitched of the object under examination in a supine posture, thereby obtaining at least two lateral X-ray images of the first body part to be stitched; and
stitching the at least two lateral X-ray images to obtain a radiograph of the first body part to be stitched.

In some embodiments, said controlling the X-ray head and the detector to cooperatively image the first body part to be stitched of the object under examination in a supine posture, thereby obtaining at least two lateral X-ray images of the first body part to be stitched comprises:
controlling the X-ray head to move to a plurality of first travel points that are distributed at the first side of the bed platform along the first direction; and when the X-ray head is moved to each of at least two first travel points, controlling the X-ray head to emit X-rays to the first body part to be stitched;
or, the X-ray head being manually moved by an operator-applied force to a plurality of first travel points that are distributed at the first side of the bed platform along the first direction; and when the X-ray head is moved to each of at least two first travel points, controlling the X-ray head to emit X-rays to the first body part to be stitched.

In some embodiments, said controlling the X-ray head and the detector to cooperatively image the first body part to be stitched of the object under examination in a supine posture, thereby obtaining at least two lateral X-ray images of the first body part to be stitched further comprises:
controlling the detector to move to a position corresponding to each first travel point where the X-ray head passes and emits X-rays and receive X-rays emitted by the X-ray head at said first travel point and penetrating the first body part to be stitched, thereby obtaining the at least two lateral X-ray images;
or, controlling the detector to remain stationary at the second side of the bed platform, and controlling the detector to receive X-rays emitted by the X-ray head at each first travel point where the X-ray head passes and penetrating the first body part to be stitched, thereby obtaining the at least two lateral X-ray images.

In some embodiments, said controlling the X-ray head and the detector to cooperatively image the first body part to be stitched of the object under examination in a supine posture, thereby obtaining at least two lateral X-ray images of the first body part to be stitched comprises:
the X-ray head being remained stationary at the first side of the bed platform,
a window of a beam-limiting window assembly being controlled to sequentially move to multiple first positions that are distributed along the first direction, and
the detector being controlled to sequentially move to multiple second positions corresponding to the multiple first positions respectively and receive X-rays penetrating the first body part to be stitched via the window at each second position, thereby obtaining the at least two lateral X-ray images.

According to a sixth aspect of the present disclosure, an imaging method for an X-ray imaging system is provided in some embodiments, wherein the the X-ray imaging system comprises an X-ray head, a detector, and a bed platform that is configured to support an object under examination who is lying in a supine posture along a first direction of the bed platform, the bed platform further has a first side and a second side that are distributed along its first direction, the first side and the second side are opposite sides of the bed platform; the imaging method comprising:
determining a current imaging mode, wherein the imaging mode comprises a first imaging mode for a first body part to be stitched and a second imaging mode for a second body part to be stitched;
when the current imaging mode is the first imaging mode, controlling the X-ray head and the detector to cooperatively image the first body part to be stitched of the object under examination in a supine posture, thereby obtaining at least two lateral X-ray images of the first body part to be stitched; wherein a radiograph of the first body part to be stitched is obtained by stitching the at least two lateral X-ray images; and
when the current imaging mode is the second imaging mode, controlling the X-ray head and the detector to cooperatively image the second body part to be stitched of the object under examination in a supine posture, thereby obtaining at least two anteroposterior X-ray images of the second body part to be stitched; wherein a radiograph of the second body part to be stitched is obtained by stitching the at least two anteroposterior X-ray images.

In some embodiments, said controlling the X-ray head and the detector to cooperatively image the first body part to be stitched of the object under examination in a supine posture, thereby obtaining at least two lateral X-ray images of the first body part to be stitched comprises:
controlling the X-ray head to move to a plurality of first travel points that are distributed at the first side of the bed platform along the first direction; and when the X-ray head is moved to each of at least two first travel points, controlling the X-ray head to emit X-rays to the first body part to be stitched;
or, the X-ray head being manually moved by an operator-applied force to a plurality of first travel points that are distributed at the first side of the bed platform along the first direction; and when the X-ray head is moved to each of at least two first travel points, controlling the X-ray head to emit X-rays to the first body part to be stitched.

In some embodiments, said controlling the X-ray head and the detector to cooperatively image the first body part to be stitched of the object under examination in a supine posture, thereby obtaining at least two lateral X-ray images of the first body part to be stitched further comprises:
controlling the detector to move to a position corresponding to each first travel point where the X-ray head passes and emits X-rays and receive X-rays emitted by the X-ray head at said first travel point and penetrating the first body part to be stitched, thereby obtaining the at least two lateral X-ray images; wherein the X-ray receiving device further comprises the second drive mechanism;
or, controlling the detector to remain stationary at the second side of the bed platform, and controlling the detector to receive X-rays emitted by the X-ray head at each first travel point where the X-ray head passes and penetrating the first body part to be stitched, thereby obtaining the at least two lateral X-ray images.

In some embodiments, said determining a current imaging mode comprises: determining the current imaging mode in response to triggering of a mode switching interface; and
the imaging method further comprises:
when the current imaging mode is the first imaging mode, controlling the X-ray head to move to the first side of the bed platform before imaging; and
when the current imaging mode is the second imaging mode, controlling the X-ray head to move to a position over the bed platform before imaging.

The X-ray imaging systems and imaging methods thereof mentioned above enable imaging of the lateral view of the object under examination in a stitching manner when the object under examination lies flat in a supine position on the bed platform.

### BRIEF DESCRIPTION OF THE DRAWINGS

FIG. 1 is an exemplary diagram showing performing imaging in a standing position;
FIG. 2 is another exemplary diagram showing performing imaging in a standing position;
FIG. 3 is an exemplary diagram showing performing imaging in a standing position;
FIG. 4 is another exemplary diagram showing performing imaging in a standing position;
FIG. 5 is a schematic structural diagram of an X-ray imaging system according to some embodiments;
FIG. 6 is a schematic structural diagram of an X-ray imaging system according to some embodiments;
FIG. 7 is a schematic structural diagram of an X-ray imaging system according to some embodiments;
FIG. 8 is a schematic structural diagram of an X-ray imaging system according to some embodiments;
FIG. 9 is a schematic structural diagram of an X-ray imaging system according to some embodiments;
FIG. 10 is a schematic structural diagram of an X-ray imaging system according to some embodiments;
FIG. 11 is a schematic structural diagram of an X-ray imaging system according to some embodiments;
FIG. 12 is a schematic diagram illustrating imaging the lateral view of a first body part to be stitched by an X-ray imaging system according to some embodiments;
FIG. 13 is a schematic diagram illustrating imaging the lateral view of a first body part to be stitched by an X-ray imaging system according to some embodiments;
FIG. 14 is a schematic diagram illustrating imaging the lateral view of a first body part to be stitched by an X-ray imaging system according to some embodiments;
FIG. 15 is a schematic structural diagram of a beam-limiting window assembly according to some embodiments;
FIG. 16 is a schematic structural diagram of an X-ray imaging system according to some embodiments;
FIG. 17 is a schematic structural diagram of an X-ray imaging system according to some embodiments;
FIG. 18 is a flowchart of an imaging method for an X-ray imaging system according to some embodiments;
FIG. 19 is a flowchart of an imaging method for an X-ray imaging system according to some embodiments;
FIG. 20 is a flowchart of an imaging method for an X-ray imaging system according to some embodiments; and
FIG. 21 is a flowchart of an imaging method for an X-ray imaging system according to some embodiments.

### DETAILED DESCRIPTION

Specific embodiments of the present disclosure will now be described in detail with reference to the accompanying drawings. Similar or related components in different embodiments are labeled with associated reference numerals. The following embodiments include detailed descriptions to facilitate understanding of the present disclosure. However, those skilled in the art will readily recognize that certain features may be omitted under specific circumstances or substituted by other components, materials, or methods. In some instances, certain operations related to the present disclosure are not explicitly described or illustrated herein. This intentional exclusion is intentional to avoid obscuring the core technical solutions of the present disclosure. For those skilled in the art, a complete understanding of these operations can be attained through the descriptions provided in this specification and general technical knowledge in the art.

Additionally, the features, operations, or characteristics described in the specification may be combined in any suitable manner to form various embodiments. Similarly, steps or actions in the method descriptions may be reordered or modified in ways that would be obvious to those skilled in the art. Therefore, the sequences presented in the specification and drawings are intended solely to clarify the description of specific embodiments and do not imply mandatory orderings, unless explicitly stated that a particular sequence is required.

The numerical designations assigned to components in this specification, such as 'first,' 'second,' or similar ordinal terms, serve solely to distinguish described objects and carry no inherent sequential or technical implications. Furthermore, the terms 'connected' and 'coupled' as used herein encompass both direct and indirect connection (coupling), unless explicitly stated otherwise.

As described above, even in supine-position imaging scenarios, an object under examination may fail to autonomously sustain the lateral decubitus posture (i.e., lateral supine posture) illustrated in FIG. 4 due to injury or medical conditions, thereby preventing successful imaging of lateral view of the object under examination. Additionally, in some clinical circumstances, single-exposure X-ray imaging cannot fully capture the exposable body part (e.g., spine or lower extremities, hereinafter referred to as "a body part to be stitched"). In such cases, it is necessary to partition the exposable body part into different zones for sequential X-ray imaging, followed by stitching of the captured images to reconstruct a complete X-ray image of the exposable body part. Consequently, when imaging the lateral view of the body part to be stitched is required for the object under examination that is incapable of maintaining the lateral decubitus posture shown in FIG. 4, this necessitates prolonged maintenance of the lateral supine posture, thereby imposing exacerbated physical challenges on the object under examination.

To address the aforementioned technical limitations, one potential solution involves modifying the bed 04 by incorporating mechanical mechanisms to assist the object under examination in maintaining the lateral decubitus posture. In some embodiments of the present disclosure, an alternative approach is provided: allowing the object under examination to lie supine on the bed 04 in a comfortable and relaxed posture while implementing structural modifications to enable the X-ray head 01 and detector 02 to be positioned at opposing lateral sides (i.e. the left and right sides) of the bed 04. This configuration facilitates lateral projection imaging of the lateral view of the object under examination while maintaining in a supine comfort state.

In some embodiments of the present disclosure, the term "body part to be stitched" (e.g., first body part to be stitched and second body part to be stitched described hereinafter) refers to a tissue area of the object under examination that requires multiple imaging exposures during X-ray examination, wherein a radiograph of said tissue area is obtained through stitching of multiple digital X-ray images. Exemplary body parts to be stitched include, but are not limited to, the thoracic region, abdominal region, or lower extremities. It should be explicitly understood that the body part to be stitched does not denote any specific physical posture of the object under examination.

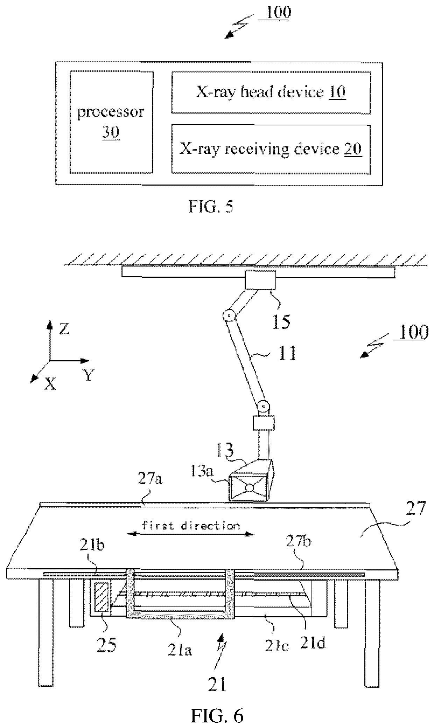

Please refer to FIGS. 5, 6, 7, and 8, which show several schematic diagrams of an X-ray imaging system 100. The X-ray imaging system 100 includes an X-ray head device 10, an X-ray receiving device 20, and a processor 30, which will be described in detail below.

In some embodiments, the X-ray head device 10 may include a support assembly 11 and an X-ray head 13. In some examples, the X-ray head device 10 may further include a first drive mechanism 15 configured to drive movement of the X-ray head 13, as will be detailed below.

In some examples, the X-ray head 13 is disposed on the first support assembly 11, which is configured to support the X-ray head 13. The first support assembly 11 may have multiple structural implementations. For example, the first support assembly 11 may adopt a ceiling-suspended structure suspended from a room ceiling.

In some examples, the X-ray head 13 is configured to emit X-rays to an object under examination. For instance, the X-ray head 13 generates and emits X-rays under high-voltage signals. In some examples, the X-ray head 13 may include a beam limiter 13a. The beam limiter 13a is configured to define a radiating area of X-rays emitted from the X-ray head 13, which may be referred to as an irradiation field or irradiation field area. Prior to imaging, an operator typically adjusts the light field (or light field area) of the X-ray head 13. Specifically, the X-ray head 13 emits visible light to simulate X-rays, wherein the visible light is constrained by the beam limiter 13a to form a projected area (i.e., the light field) that mimics the X-ray irradiation field (i.e., the irradiation field) during imaging. This configuration enables the operator to intuitively adjust the size and position of the light field prior to imaging, thereby achieving adjustment of the X-ray irradiation field during the imaging process.

In some embodiments, to accommodate imaging of objects with different heights or different body parts, the X-ray head 13 is movably disposed on the first support assembly 11. For example, an operator may manually apply force to move the X-ray head 13. For another example, the operator may issue commands to the processor 30 via functional buttons, and when the processor 30 controls the first drive mechanism 15 to drive movement of the X-ray head 13. In some examples, such movement includes translation and/or rotation.

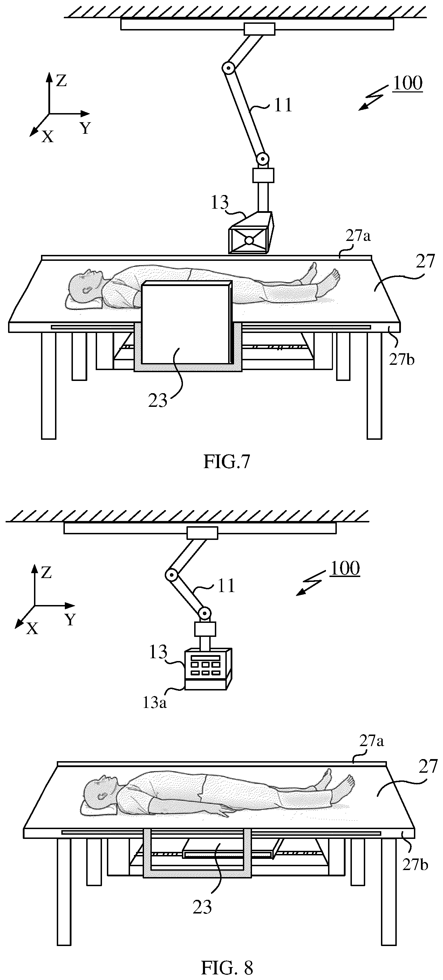

In some embodiments, the X-ray head 13 is capable of being positioned via the first support assembly 11 at a first side 27a of a bed platform 27. For example, FIG. 7 illustrates an example where the X-ray head 13 is capable of being positioned via the first support assembly 11 at the first side 27a of the bed platform 27, and the X-ray head 13 performs imaging from the negative X-axis direction shown in the figure toward the positive X-axis direction (or from the left side to the right side of the bed platform 27), such as capturing lateral views (sagittal plane) of an object under examination lying horizontally along a first direction as shown.

In some embodiments, the X-ray head 13 is capable of being positioned via the first support assembly 11 above the bed platform 27. For example, FIG. 8 illustrates an example where the X-ray head 13 is capable of being positioned via the first support assembly 11 above the bed platform 27, and the X-ray head 13 performs imaging from the positive Z-axis direction toward the negative Z-axis direction (or from top to bottom), such as capturing anterior-posterior views (coronal plane) of an object under examination lying horizontally along the first direction as shown.

The above describes some implementations of the X-ray head device 10.

In some embodiments, the X-ray receiving device 20 includes a second support assembly 21, a detector 23, and a bed platform 27. In some examples, the X-ray receiving device 20 may further include a second drive mechanism 25 for driving movement of the detector 23. Details are provided below.

In some examples, the bed platform 27 is configured to support an object under examination. For instance, the bed platform 27 is configured to support the object under examination to lie flat in a supine posture along the first direction of the bed platform 27. The bed platform 27 further includes a first side 27a and a second side 27b that are distributed along its first direction, with the first side 27a and the second side 27b being opposing sides of the bed platform 27. As shown in the figures, the Z-axis direction corresponds to the vertical direction, and the XY-plane corresponds to the horizontal plane. The first direction aligns with the Y-axis direction in the figures. Thus, the bed platform 27 allows the object under examination to lie flat in a supine posture along its Y-axis direction, such that the longitudinal axis (or vertical axis) of the object under examination remains parallel to the Y-axis direction. The bed platform 27 further has the first side 27a and second side 27b that are distributed along the Y-axis direction; that is, the first side 27a and the second side 27b are also distributed along the Y-axis direction. When the object under examination lies flat along the Y-axis direction of the bed platform 27, the first side 27a and second side 27b are positioned at the left and right sides of the object under examination, respectively, representing the two lateral edges of the bed platform 27. Additionally, as illustrated, the bed platform 27 has a rectangular shape, making the first direction coincident with the longitudinal axis direction of the bed platform. The first side 27a and second side 27b correspond to the left and right lateral edges of the bed platform 27-for clarity, if the first side 27a is the left edge, the second side 27b is the right edge, and vice versa.

In some examples, the detector 23 is configured to receive X-rays penetrating the object under examination. The detector 23 critically influences imaging quality by receiving X-rays, converting them into visible light, and further converting the visible light into electrical signals to complete image data acquisition for X-ray imaging.

In some embodiments, the second support assembly 21 is configured to support the detector 23.

In some examples, to accommodate imaging of different body parts, the detector 23 is movably mounted on the second support assembly 21. For instance, an operator may manually apply force to move the detector 23. For another instance, the operator may issue commands to a processor 30 via functional buttons, whereafter the processor 30 controls the second drive mechanism 25 to drive movement of the detector 23. In some examples, such movement may include translation and/or rotation.

In some embodiments, the detector 23 is capable of being positioned at the second side 27b of the bed platform 27 via the second support assembly 21. For example, as shown in FIG. 7, the detector 23 is capable of being positioned at the second side 27b of the bed platform 27 via the second support assembly 21, and the detector 23 receives X-rays emitted to the lateral view (sagittal plane) of the object under examination from the negative X-axis direction to the positive X-axis direction (i.e., from the left to the right side of the bed platform 27), thereby obtaining a lateral X-ray image of the object under examination.

In some examples, the second support assembly 21 includes a mounting bracket 21a configured to detachably mount the detector 23. The mounting bracket 21a is disposed at the second side 27b of the bed platform 27 and is movable along the first direction (e.g., along the positive and negative Y-axis directions as depicted in the figures), enabling the detector 23 to be capable of being positioned at the second side 27b of the bed platform 27 and be driven to move with the mounting bracket 21a. In some examples, the second support assembly 21 may further include a track 21b extending along the second side 27b of the bed platform 27. The mounting bracket 21a is slidably coupled to the track 21b, enabling the mounting bracket 21a to slide along the second side 27b of the bed platform 27. For instance, the mounting bracket 21a is driven by a second drive mechanism 25 to move along the track 21b at the second side 27b of the bed platform 27.

In some embodiments, the detector 23 is capable of being positioned below the bed platform 27 via the second support assembly 21. For example, as shown in FIG. 8, the detector 23 is positioned beneath the bed platform 27 via the second support assembly 21, and the detector 23 receives X-rays emitted to the anteroposterior view (coronal plane) of the object under examination along the positive-to-negative Z-axis direction (i.e., from top to bottom) as shown, thereby generating an anteroposterior X-ray image of the object under examination.

In some examples, the second support assembly 21 includes a carrier bracket 21c configured to support the detector 23. The carrier bracket 21c is connected to the bed platform 27 and positioned beneath the bed platform 27. The carrier bracket 21c has a space to carry or accommodate the detector 23. In some examples, a sliding rail 21d may be arranged within this space. The sliding rail 21d may include a securing structure to secure the detector 23, enabling the detector 23 to move along the sliding rail 21d. For instance, the detector 23 may be driven to move along the sliding rail 21d in the first direction.

The above describes exemplary configurations of the X-ray receiving device 20.

The X-ray head 13 and the detector 23 can cooperate to image the object under examination. The following provides detailed explanations.

The imaging of the lateral view of the object under examination is described below, referred to as a first imaging mode.

In some embodiments, the X-ray head 13 is positioned at a first side 27a of the bed platform 27 via a first support assembly 11, while the detector 23 is positioned at a second side 27b of the bed platform 27 via a second support assembly 21. This configuration allows the X-ray head 13 and the detector 23 to face toward the lateral view of the object under examination lying flat in a supine posture on the bed platform 27 to acquire a lateral X-ray image of the object under examination.

In some embodiments, the processor 30 acquires a first body part to be stitched of the object under examination; in response to an exposure instruction for the first body part to be stitched, the processor 30 controls the X-ray head 13 and the detector 23 to cooperatively image the first body part to be stitched of the object under examination in a supine posture, obtaining at least two lateral X-ray images of the first body part to be stitched; and the processor 30 stitches these at least two lateral X-ray images to obtain a radiograph of the first body part to be stitched.

In some embodiments, the first imaging mode is used to image the first body part to be stitched. When the current imaging mode is the first imaging mode, the processor 30 controls the X-ray head 13 and the detector 23 to cooperatively image the first body part to be stitched of the object under examination in a supine posture, acquiring at least two lateral X-ray images of the first body part to be stitched. These at least two lateral X-ray images are stitched to produce the radiograph of the first body part to be stitched.

As shown in FIG. 7, during the first imaging mode, the X-ray head 13 and the detector 23 cooperate to image the lateral view of the object under examination in a supine posture on the bed platform 27 to acquire the lateral X-ray image of the object under examination. During imaging, the X-ray head 13 is positioned at the first side 27a of the bed platform 27 via the first support assembly 11, and the detector 23 is positioned at the second side 27b of the bed platform 27 via the second support assembly 21, while the object under examination lies supine along the first direction on the bed platform 27; in this way, the object under examination is positioned between the X-ray head 13 and the detector 23, i.e., the X-ray head 13 and the detector 23 are located at opposite lateral sides of the object under examination. This configuration enables cooperative imaging of the lateral view of the object under examination.

To expedite imaging, a quick positioning method may be provided before imaging to rapidly position the X-ray head 13 and/or the detector 23 to the first side 27a and second side 27b.

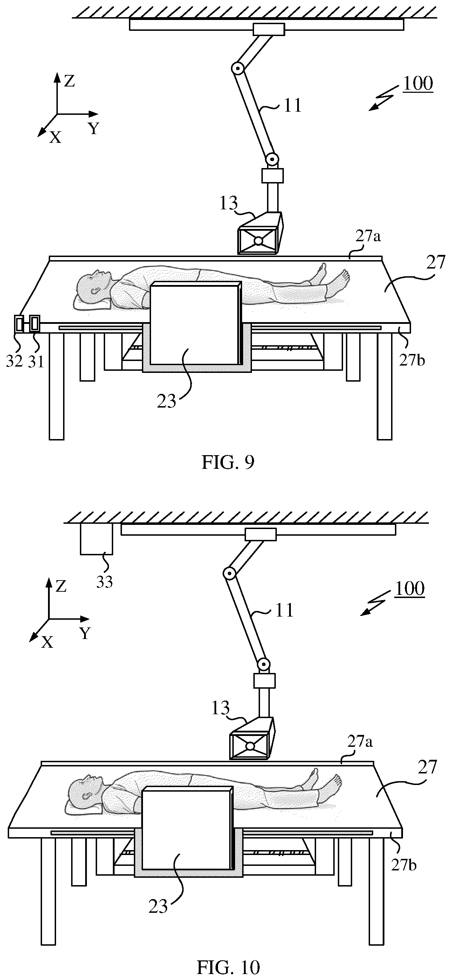

As shown in FIG. 9, in some embodiments, the X-ray imaging system 100 further includes a first positioning button 31 and/or a second positioning button 32, as described below.

In some embodiments, the first positioning button 31 can be triggered by an operator to instruct the processor 30 to control a first drive mechanism 11 to move the X-ray head 13 to the first side 27a of the bed platform 27.

The first positioning button 31 may be a physical functional button, wherein the form of the physical functional button may include, but is not limited to, button-type, knob-type, or toggle lever-type configurations. The first positioning button 31 may also be a virtual functional button, wherein the form of the virtual functional button may include an operational control element on a display interface, which can be presented as a menu or a simulated physical control element mimicking an actual key. The first positioning button 31 may be disposed on the bed platform 27, the second support assembly 2, or the X-ray head 13.

In some embodiments, the second positioning button 32 can be triggered by an operator to instruct the processor 30 to control a second drive mechanism 21 to move the detector 23 to the second side 27b of the bed platform 27.

The second positioning button 32 may be a physical functional button, wherein the form of the physical functional button may include, but is not limited to, button-type, knob-type, or toggle lever-type configurations. The second positioning button 32 may also be a virtual functional button, wherein the form of the virtual functional button may include an operational control element on a display interface, which can be presented as a menu or a simulated physical control element mimicking an actual key. The second positioning button 32 may be disposed on the bed platform 27, the second support assembly 2, or the X-ray head 13.

The first positioning button 31 and the second positioning button 32 respectively trigger the X-ray head 13 to move to the first side 27a of the bed platform 27 and the detector 23 to move to the second side 27b of the bed platform 27. To further optimize operational efficiency and time savings, the first positioning button 31 may be triggered to drive the X-ray head 13 to a first positioning point at the first side 27a of the bed platform 27, while the second positioning button 32 may be triggered to drive the detector 23 to a second positioning point at the second side 27b of the bed platform 27, wherein the first positioning point and the second positioning point are mutually aligned such that the X-ray head 13 and the detector 23 satisfy a positional matching relationship required before imaging. In some example, the positional matching relationship required before imaging include the X-ray head 13 and the detector 23 meeting a predetermined distance requirement and/or a predetermined angular requirement. For example, the positional matching relationship may require the X-ray head 13 to be directly opposed to the detector 23, wherein an emission axis of the X-ray head 13 passes through the center of the detector 23 and is perpendicular to a plane where the detector 23 locates. It is noted that the X-ray emitted by the X-ray head 13 is generally a conical X-ray beam, and the emission axis of the X-ray head 13 refers to the central axis of the conical X-ray beam.

In some examples, the first positioning button 31 and the second positioning button 32 are two distinct functional buttons.

In some examples, the first positioning button 31 and the second positioning button 32 are implemented as a single shared functional button. In such configurations, triggering of this shared functional button enables an operator to cause the processor 30 to control the first drive mechanism 11 to drive the X-ray head 13 to the first side 27a of the bed platform 27, and control the second drive mechanism 21 to drive the detector 23 to the second side 27b of the bed platform 27.

The above describes implementation via the functional button(s) to rapid pre-imaging positioning of the X-ray head 13 and the detector 23 for the first imaging mode.

Please refer to FIG. 10. In some embodiments, the X-ray imaging system 100 further includes an image capturing device 33. In some embodiments, the image capturing device 33 is configured to capture a first image that comprises an optical image with two-dimensional spatial information and/or a depth image with three-dimensional spatial information.

In some embodiments, the processor 30 is further configured to: perform a first path planning based on the first image to control the first driving mechanism 11 to drive the X-ray head 13 to move to the first side 27a of the bed platform 27. In some examples, the processor 30 is further configured to: perform a second path planning based on the first image to control the second driving mechanism 21 to drive the detector 23 to move to the second side 27b of the bed platform 27. In some examples, the processor 30 performs the first path planning based on the first image to control the first driving mechanism 11 to drive the X-ray head 13 to the first positioning point at the first side 27a of the bed platform 27, and performs the second path planning based on the first image to control the second driving mechanism 21 to drive the detector 23 to the second positioning point at the second side 27b of the bed platform 27, wherein the first positioning point and the second positioning point are matched such that the X-ray head 13 and the detector 23 satisfy a positional matching relationship required before imaging. In some examples, the positional matching relationship required before imaging includes the X-ray head 13 and the detector 23 meeting a predetermined distance requirement and/or a predetermined angular requirement. For example, the positional matching relationship may require the X-ray head 13 to be directly opposed to the detector 23, meaning an emission axis of the X-ray head 13 passes through a center of the detector 23 and is perpendicular to a plane where the detector 23 locates. It should be noted that the X-rays emitted from the X-ray head 13 generally form a conical X-ray beam, and the emission axis of the X-ray head 13 refers to the central axis of the conical X-ray beam.

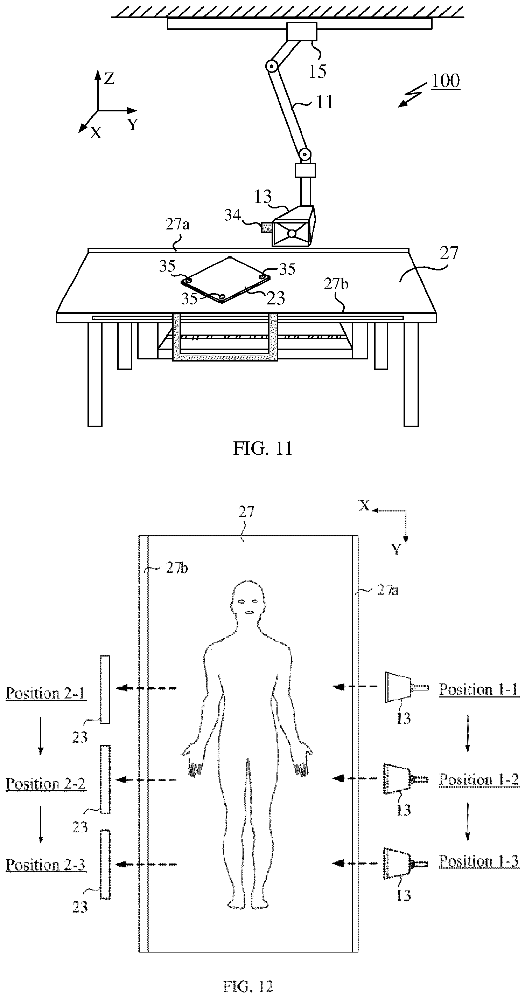

Please refer to FIG. 11, in some embodiments, the X-ray imaging system 100 further includes at least one base station 34 disposed on the X-ray head 13 and at least one tag 35 disposed on the detector 23. The base station 34 is configured to transmit signals to the tag 35 and receive signals returned from the tag 35.

In some embodiments, the processor 30 is further configured to: calculate relative position information between the base station 34 and the tag 35 based on signals transmitted and received by the base station 34, and control the first driving mechanism 11 to drive the X-ray head 13 to move based on the relative position information, such that the X-ray head 13 and the detector 23 satisfy a positional matching relationship required before imaging. In some examples, the positional matching relationship required before imaging includes the X-ray head 13 and the detector 23 meeting a predetermined distance requirement and/or a predetermined angular requirement. For example, the positional matching relationship may require the X-ray head 13 to be directly opposed to the detector 23, meaning an emission axis of the X-ray head 13 passes through a center of the detector 23 and is perpendicular to a plane where the detector 23 locates. It should be noted that the X-rays emitted from the X-ray head 13 generally form a conical X-ray beam, and the emission axis of the X-ray head 13 refers to the central axis of the conical X-ray beam.

In this way, an operator first places the detector 23 at the second side 27b of the bed platform 27, and the processor 30 is then further configured to: calculate relative position information between the base station 34 and the tag 35 based on signals transmitted and received by the base station 34, and control the first driving mechanism 11 to drive the X-ray head 13 to move based on the relative position information, such that the X-ray head 13 and the detector 23 satisfy the positional matching relationship required before imaging.

The base station 34 and the tag 35 may be configured in a one-to-one or one-to-many relationship. For a base station 34, by transmitting signals from the base station 34 to a tag 35 and receiving signals returned from the tag 35, the processor 30 is configured to compute relative position information between the base station 34 and the tag 35. Accordingly, when the spatial position of either the base station 34 or the tag 35 is known, the spatial position of the other can be determined using the computed relative position information. As used herein, "spatial position" refers to the location of an object in three-dimensional space, which may be described using an established XYZ three-dimensional coordinate system. It is understood that the origin of the XYZ three-dimensional coordinate system may be set at any location based on practical requirements. In some embodiments, the relative position information between an object A and an object B includes a relative distance and a relative angular orientation. Specifically: the relative distance between the object A and the object B is defined as the distance between two points representing the object A and the object B; and the relative angular orientation between the object A and the object B is defined as the angular orientation, in three-dimensional space, of a line defined by the two points representing the object A and the object B, wherein this angular orientation may be measured relative to the X-axis, Y-axis, and/or Z-axis of the XYZ three-dimensional coordinate system. In this way, when the relative position information between the object A and the object B is known, and the spatial position of either the object A or the object B is known, the spatial position of the other can be determined. Accordingly, the relative position information between the base station 34 and the tag 35 includes a relative distance and a relative angular orientation. Specifically: the relative distance between the base station 34 and the tag 35 is defined as the distance between two points representing the base station 34 and the tag 35; and the relative angular orientation between the base station 34 and the tag 35 is defined as the angular orientation, in three-dimensional space, of a line defined by the two points representing the base station 34 and the tag 35, wherein this angular orientation may be measured relative to the X-axis, Y-axis, and/or Z-axis of the XYZ three-dimensional coordinate system. In this way, when the relative position information between the base station 34 and the tag 35 is known, and the spatial position of either the base station 34 or the tag 35 is known, the spatial position of the other can be determined.

In some embodiments, three tags 35 are disposed on the detector 23, with each tag 35 positioned at one of three corners of the detector 23 to define a plane.

In some embodiments, the processor 30 is configured to calculate the spatial position of the detector 23 based on the relative position information between the base station 34 and the tag 35.

In some embodiments, the processor 30 is configured to calculate orientation information of the detector 23 based on the relative position information between the base station 34 and the tag 35.

Additionally, since the processor 30 can control the first driving mechanism 11 to drive the X-ray head 13 to move, the processor 30 can obtain the spatial position and even orientation information (e.g., directional alignment) of the X-ray head 13 via the first driving mechanism 11.

In some examples, the detector 23 is a portable flat-panel detector.

By means of the aforementioned configurations, rapid pre-imaging positioning of the X-ray head 13 and the detector 23 for the first imaging mode can be achieved prior to imaging.

When performing imaging of the lateral view of the first body part to be stitched, the X-ray head 13 and the detector 23 are required to collaboratively execute multiple imaging on the lateral view of the first body part to be stitched. The following describes the coordination mechanism between the X-ray head 13 and the detector 23.

In some embodiments, the processor 30 controls the first driving mechanism 15 to drive the X-ray head 13 to move to multiple first travel points distributed along a first direction at the first side 27a of the bed platform 27; and when the X-ray head 13 reaches each of at least two first travel points, the processor 30 controls the X-ray head 13 to emit X-rays to the first body part to be stitched. In some embodiments, the first travel points are exposure points where the X-ray head emits the X-rays to the body part to be stitched.

In some embodiments, the X-ray head 13 is manually moved by an operator applying force to multiple first travel points distributed along the first direction at the first side 27a of the bed platform 27; and when the X-ray head 13 reaches each of at least two first travel points, the processor 30 controls the X-ray head 13 to emit X-rays to the first body part to be stitched.

The above describes some operational movements of the X-ray head 13 during imaging. The following details how the detector 23 coordinates therewith.

In some embodiments, the processor 30 controls the second driving mechanism 21 to drive the detector 23 to move to a position corresponding to each first travel point that the X-ray head 13 passes and where the X-rays are emitted, and receive X-rays emitted from the X-ray head 13 at that first travel point and penetrating the first body part to be stitched, thereby obtaining the aforementioned at least two lateral X-ray images.

FIG. 12 illustrates an example, where the orientations of the coordinate system in FIG. 12 aligns with that in FIG. 7. FIG. 12 represents a top-down view from the positive Z-axis direction toward the negative Z-axis direction (i.e., viewed from above), with the Y-axis direction designated as the first direction. On the first side 27a of the bed platform 27, three first travel points are distributed along the first direction: Position 1-1, Position 1-2, and Position 1-3 as shown. The detector 23 is correspondingly positioned at Position 2-1, Position 2-2, and Position 2-3, which respectively align with the X-ray head 13 at Position 1-1, Position 1-2, and Position 1-3. Initially, the X-ray head 13 is positioned at Position 1-1, and the detector 23 is at Position 2-1 corresponding to Position 1-1. The X-ray head 13 emits X-rays from the negative X-axis direction to the positive X-axis direction, and the detector 23 at Position 2-1 receives X-rays penetrating through the lateral view of the object under examination, thereby acquiring a lateral X-ray image. Similarly, the X-ray head 13 is moved from Position 1-1 to Position 1-2, and the detector 23 is moved from Position 2-1 to Position 2-2. At Position 1-2, the X-ray head 13 emits X-rays from the negative X-axis direction to the positive X-axis direction, while the detector 23 at Position 2-2 receives X-rays penetrating through the lateral view of the object under examination, acquiring another lateral X-ray image. Further, the X-ray head 13 is moved from Position 1-2 to Position 1-3, and the detector 23 is moved from Position 2-2 to Position 2-3. At Position 1-3, the X-ray head 13 emits X-rays from the negative X-axis direction to the positive X-axis direction, and the detector 23 at Position 2-3 receives X-rays penetrating through the lateral view of the object under examination, obtaining yet another lateral X-ray image. It is noted that the dashed-line arrows in the figure are schematic representations of the X-ray beams emitted by the X-ray head 13.

In some embodiments, the detector 23 remains stationary at the second side 27b of the bed platform 27; for example, the processor 30 controls the detector 23 to stay stationary at the second side 27b of the bed platform 27; and the processor 30 controls the detector 23 to receive X-rays emitted by the X-ray head 13 at each first travel point that the X-ray head 13 passes and where the X-rays are emitted and penetrating the first body part to be stitched, thereby obtaining the aforementioned at least two lateral X-ray images.

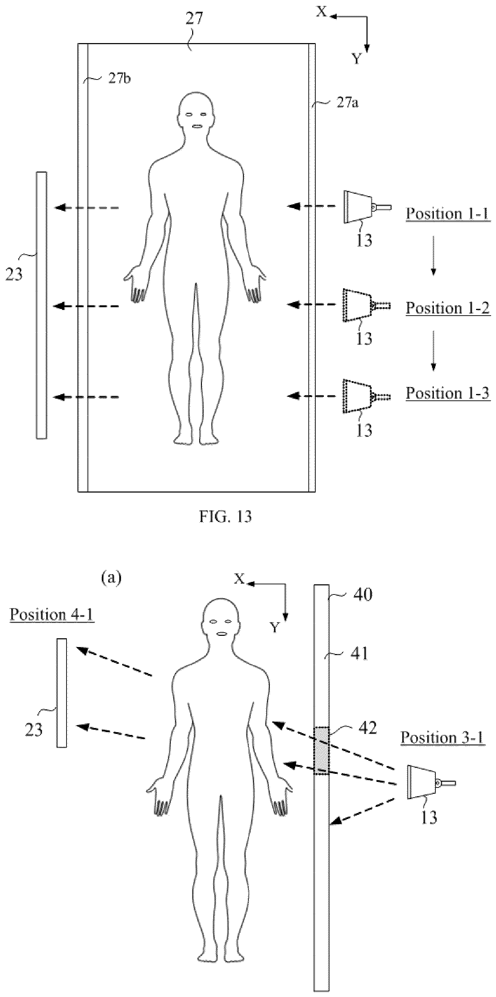

FIG. 13 illustrates an example, where the orientations of the coordinate system in FIG. 13 aligns with that in FIG. 7. FIG. 13 represents a top-down view from the positive Z-axis direction toward the negative Z-axis direction (i.e., viewed from above), with the Y-axis direction designated as the first direction. On the first side 27a of the bed platform 27, three first travel points are distributed along the first direction: Position 1-1, Position 1-2, and Position 1-3 as shown. The detector 23 is correspondingly positioned at Position 2-1, Position 2-2, and Position 2-3, while the detector 23 remains stationary without movement. Initially, the X-ray head 13 is positioned at Position 1-1; the X-ray head 13 emits X-rays from the negative X-axis direction to the positive X-axis direction, and the detector 23 receives X-rays penetrating through the lateral view of the object under examination, thereby acquiring a lateral X-ray image. Subsequently, the X-ray head 13 is moved from Position 1-1 to Position 1-2, and at Position 1-2, the X-ray head 13 emits X-rays from the negative X-axis direction to the positive X-axis direction, while the detector 23 receives X-rays penetrating through the lateral view of the object under examination, acquiring another lateral X-ray image. Similarly, the X-ray head 13 is moved from Position 1-2 to Position 1-3, and at Position 1-3, the X-ray head 13 emits X-rays from the negative X-axis direction to the positive X-axis direction, and the detector 23 receives X-rays penetrating through the lateral view of the object under examination, obtaining yet another lateral X-ray image. It is noted that the dashed-line arrows in the figure are schematic representations of the X-ray beams emitted by the X-ray head 13.

In examples where the detector 23 remains stationary to cooperate with the X-ray head 13, the detector 23 is designed with a relatively large size. This configuration enables the detector 23 to capture X-rays emitted by the X-ray head 13 at respective first travel point and penetrating through the lateral view of the object under examination.

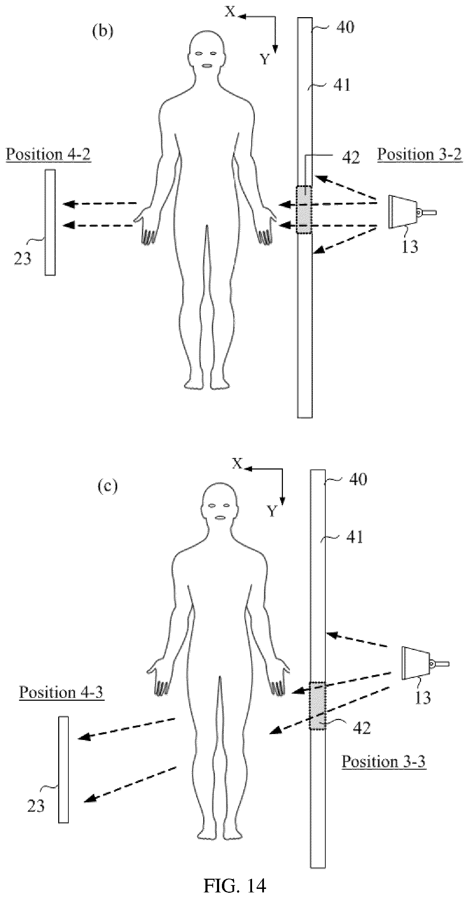

As shown in FIG. 14, the X-ray imaging system 100 further includes a beam-limiting window assembly 40 positioned between the first body part to be stitched and the X-ray head 13. The beam-limiting window assembly 40 comprises a shielding portion 41 for blocking X-rays and a window 42 defined by the shielding portion 41, wherein the window 42 allows X-rays to pass through. In some examples, the X-ray head 13 emits a conical X-ray beam symmetric about the emission axis of the X-ray head. The window 42 allows transmission of a portion of the conical X-ray beam during each imaging exposure. In some examples, the window 42 enables formation of a non-axisymmetric X-ray beam during at least one imaging exposure, where said non-axisymmetric X-ray beam constitutes an X-ray beam lacking symmetry about the aforementioned emission axis after partial transmission of the conical X-ray beam through the window.

In some embodiments, the beam-limiting window assembly 40 and the X-ray head 13 are capable of independent movement, as they are mechanically separate structures. This means that movement of the X-ray head 13 does not drive synchronized motion of the beam-limiting window assembly 40.

Referring to FIG. 14(a), (b), and (c), the coordinate system orientation defined in FIG. 14 aligns with those established in other figures described above, which will not be reiterated herein. During imaging operations, the processor 30 controls the X-ray head 13 stationary while commanding it to emit conical X-ray beams with identical irradiation fields during each exposure. The illustration depicts three first positions designated as Position 3-1, Position 3-2, and Position 3-3, with corresponding three second positions i.e. Position 4-1, Position 4-2, and Position 4-3 respectively. The processor 30 controls the window 42 to move to Position 3-1 and the detector 23 to Position 4-1; and at position 4-1, the processor 30 controls the detector 23 to receive X-rays penetrating through the first body part to be stitched via the window 42 at Position 3-1, thereby acquiring a lateral X-ray image. Similarly, the processor 30 controls the window 42 to move to Position 3-2 and the detector 23 to Position 4-2; and at Position 4-2, the processor 30 controls the detector 23 to receive X-rays penetrating through the first body part to be stitched via the window 42 at Position 3-2, obtaining another lateral X-ray image. Similarly, the processor 30 controls the window 42 to move to Position 3-3 and the detector 23 to Position 4-3; and at Position 4-3, the processor 30 controls the detector 23 to receive X-rays penetrating through the first body part to be stitched via the window 42 at Position 3-3, obtaining yet another lateral X-ray image.

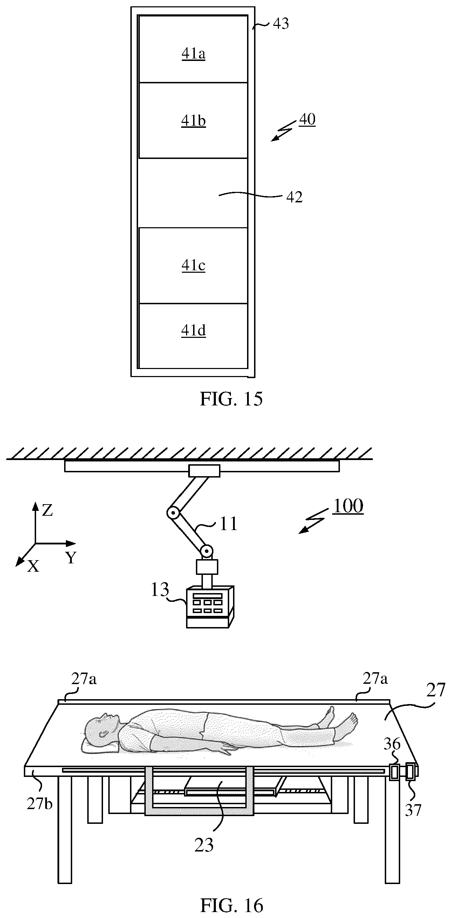

Please refer to FIG. 15, in some embodiments, the beam-limiting window assembly 40 includes a support frame 43. The shielding portion 41 comprises shielding plates 41a, 41b, 41c, and 41d. The shielding plates 41a and 41d are fixedly disposed on the support frame 43 to define a first window. The shielding plates 41b and 41c are slidably arranged on the support frame 43 to delineate a second window from the first window as the aforementioned window 42. The processor 40 controls the sliding of the shielding plates 41b and 41c on the support frame 43 to enable movement of the second window (i.e., window 42) further defined by the shielding plates 41b and 41c within the first window.

The beam-limiting window assembly 40 enables shielding of unnecessary X-rays during multiple exposures of the first body part to be stitched, thereby reducing radiation exposure to the object under examination.

To determine coordination between the X-ray head 13 and the detector 23, the processor 30 acquires the first body part to be stitched of the object under examination and determines an exposure parameter set of the first body part to be stitched. In some examples, the exposure parameter set includes: multiple first travel points that are distributed along the first direction at the first side 27a of the bed platform 27 and where the X-ray head 13 is controlled to move, and exposure parameters for the X-ray head 13 at at least two first travel points. In some embodiments, the exposure parameters include an irradiation field area.

In examples where the X-ray imaging system 100 includes an image capturing device 33, the imaging device 33 is configured to capture a second image comprising an optical image with two-dimensional spatial information and/or a depth image with three-dimensional spatial information. The processor 30 determining the exposure parameter set of the first body part to be stitched includes: the processor 30 obtaining a current positional relationship between the first body part to be stitched and the detector 23 based on the second image, and determining the exposure parameter set of the first body part to be stitched based on this current positional relationship.

In examples where the X-ray imaging system 100 includes the base station 34 and the tag 35, the processor 30 determining the exposure parameter set of the first body part to be stitched includes: the processor 30 acquiring the current positional relationship between the first body part to be stitched and the detector 23 according to the relative position information, and determining the exposure parameter set of the first body part to be stitched based on this current positional relationship.

In some examples, the processor 30 determining the exposure parameter set of the first body part to be stitched includes: the processor 30 acquiring a predetermined positional relationship between the first body part to be stitched and the detector 23 when the object under examination is in a supine posture, and determining the exposure parameter set of the first body part to be stitched based on this predetermined positional relationship.

In some examples, a body part to be stitched (e.g., the first body part to be stitched) is preconfigured with its corresponding exposure parameter set; the object under examination is guided by an operator to carry out corresponding positioning, and the processor 30 controls the X-ray head 13 and the detector 23 to execute imaging of the body part to be stitched of the object under examination according to the preconfigured exposure parameter set corresponding to the current body part to be stitched.

In some embodiments, in response to the exposure instruction for the first body part to be stitched, the processor 30 controls the X-ray head 13 and the detector 23 to cooperate according to the exposure parameter set to image the first body part to be stitched of the object under examination in a supine posture, thereby obtaining at least two lateral X-ray images of the first body part to be stitched.

For details of how the processor 30 controls cooperation between the X-ray head 13 and the detector 23 according to the exposure parameter set, reference may be made to the above descriptions. For example, the processor 30 controls the first drive mechanism 15 to move the X-ray head 13 to pass each of the at least two of the first travel points according to the exposure parameter set, and when the X-ray head 13 reaches said each of the at least two first travel point, the processor 30 controls the X-ray head 13 to emit X-rays to the first body part to be stitched of the object under examination in a supine posture on the bed platform 27 according to the exposure parameters for that first travel point; and the processor 30 controls the detector 23 at the second side 27b of the bed platform 27 to receive X-rays penetrating the first body part to be stitched, thereby obtaining at least two lateral X-ray images of the first body part to be stitched.

After acquiring the at least two lateral X-ray images, the processor 30 stitches these at least two lateral X-ray images to obtain a radiograph of the first body part to be stitched. It should be understood that during image stitching, the processor may, after obtaining all lateral X-ray images of the first body part to be stitched (i.e., after imaging exposure), stitch these lateral X-ray images to obtain the radiograph of the first body part to be stitched. When performing image stitching, the processor may alternatively execute stitching during the imaging process, wherein image stitching can be performed immediately upon acquiring a single lateral X-ray image, enabling concurrent operation of both image stitching and image acquisition procedures.

In some embodiments, the processor 30 is further configured to: acquire a region of interest (ROI) of the first body part to be stitched. The processor 30 stitching the at least two lateral X-ray images to obtain the radiograph of the first body part to be stitched includes: determining a stitching region between adjacent lateral X-ray images based on the ROI, ensuring that the ROI of the first body part to be stitched resides outside the stitching region.

It should be understood that the stitching region refer to an area formed between adjacent X-ray images when constructing the radiograph of a body part to be stitched from multiple X-ray images.

In some embodiments, the processor 30 is further configured to: acquire the ROI of the first body part to be stitched, and determine the exposure parameter set based on the ROI, for example, determining first travel points associated with the ROI to ensure complete imaging of the ROI during imaging exposure while maintaining the ROI outside the stitching region. In other words, the processor 30 determines the exposure parameter set of the first body part to be stitched to ensure the ROI is fully captured during X-ray imaging and remains outside the stitching region.

The above describes implementations of imaging of lateral view of the object under examination (or the first imaging mode). It can be seen that through the above embodiments, imaging of lateral view of the object under examination can be completed when the object under examination lies supine on the bed platform 27.

The imaging of the anteroposterior view of the object under examination is described below, referred to as a second imaging mode.

In some embodiments, the X-ray head 13 is capable of being positioned above the bed platform 27 via the first support assembly 11, and the detector 23 is capable of being positioned below the bed platform 27 via the second support assembly 21, enabling the X-ray head 13 and the detector 23 to face toward the anteroposterior view of the object under examination in a supine posture on the bed platform 27 to an anteroposterior X-ray image of the object under examination.

In some embodiments, the processor 30 acquires a second body part to be stitched of the object under examination; in response to an exposure instruction for the second body part to be stitched, the processor 30 controls the X-ray head 13 and the detector 23 to cooperatively image the second body part to be stitched of the object under examination in a supine posture, thereby obtaining at least two anteroposterior X-ray images of the second body part to be stitched; and the processor 30 stitches the at least two anteroposterior X-ray images to generate a radiograph of the second body part to be stitched.

In some embodiments, the second imaging mode is used to image the second body part to be stitched; and when the current imaging mode is the second imaging mode, the processor 30 controls the X-ray head 13 and the detector 23 to cooperatively image the second body part to be stitched of the object under examination in a supine posture, thereby obtaining at least two anteroposterior X-ray images of the second body part to be stitched. The at least two anteroposterior X-ray images are used to be stitched to produce the radiograph of the second body part to be stitched.

Please refer to the schematic diagram of FIG. 8 above. In the second imaging mode, the X-ray head 13 and the detector 23 cooperate to image anterior-posterior view of the object under examination in a supine posture on the bed platform 27, thereby obtaining the anteroposterior X-ray image of the object under examination. During imaging, the X-ray head 13 is positioned above the bed platform 27 via the first support assembly 11, the detector 23 is positioned below the bed platform 27 via the second support assembly 21, and the object under examination lies supine along the first direction on the bed platform 27, thus the object under examination is positioned between the X-ray head 13 and the detector 23 (i.e., the X-ray head 13 and the detector 23 are located above and below the object under examination, respectively). In this way, the X-ray head 13 and the detector 23 can be cooperatively image the anteroposterior view of the object under examination.

To expedite imaging, a rapid positioning method may be provided before imaging to quickly position the X-ray head 13 and/or the detector 23 above and below the bed platform 27, respectively.

As shown in FIG. 16, in some embodiments, the X-ray imaging system 100 further includes a third positioning button 36 and/or a fourth positioning button 37, as described below.

In some embodiments, the third positioning button 36 can be triggered by an operator to cause the processor 30 to control the first drive mechanism 11 to move the X-ray head 13 to a position over the bed platform 27.

The third positioning button 36 may be a physical functional button, wherein the form of the physical functional button may include, but is not limited to, button-type, knob-type, or toggle lever-type configurations. The third positioning button 36 may also be a virtual functional button, wherein the form of the virtual functional button may include an operational control element on a display interface, which can be presented as a menu or a simulated physical control element mimicking an actual key. The third positioning button 36 may be disposed on the bed platform 27, the second support assembly 2, or the X-ray head 13.

In some embodiments, the fourth positioning button 37 may be triggered by an operator to cause the processor 30 to control the second drive mechanism 21 to move the detector 23 to a position below the bed platform 27.

The fourth positioning button 37 may be a physical functional button, wherein the form of the physical functional button may include, but is not limited to, button-type, knob-type, or toggle lever-type configurations. The fourth positioning button 37 may also be a virtual functional button, wherein the form of the virtual functional button may include an operational control element on a display interface, which can be presented as a menu or a simulated physical control element mimicking an actual key. The fourth positioning button 37 may be disposed on the bed platform 27, the second support assembly 2, or the X-ray head 13.

The third positioning button 36 and the fourth positioning button 37 respectively trigger the X-ray head 13 to move to a position over the bed platform 27 and the detector 23 to move to a position below the bed platform 27. To further optimize operational efficiency and time savings, the third positioning button 36 may be triggered to drive the X-ray head 13 to a third positioning point above the bed platform 27, while the fourth positioning button 37 may be triggered to drive the detector 23 to a fourth positioning point below the bed platform 27, wherein the third positioning point and the fourth positioning point are mutually aligned such that the X-ray head 13 and the detector 23 satisfy a positional matching relationship required before imaging. In some example, the positional matching relationship required before imaging include the X-ray head 13 and the detector 23 meeting a predetermined distance requirement and/or a predetermined angular requirement. For example, the positional matching relationship may require the X-ray head 13 to be directly opposed to the detector 23, wherein an emission axis of the X-ray head 13 passes through the center of the detector 23 and is perpendicular to a plane where the detector 23 locates. It is noted that the X-ray emitted by the X-ray head 13 is generally a conical X-ray beam, and the emission axis of the X-ray head 13 refers to the central axis of the conical X-ray beam.

In some examples, the third positioning button 36 and the fourth positioning button 37 are two distinct functional buttons.

In some examples, the third positioning button 36 and the fourth positioning button 37 are implemented as a single shared functional button. In such configurations, triggering of this shared functional button enables an operator to cause the processor 30 to control the first drive mechanism 11 to drive the X-ray head 13 to move to a position over the bed platform 27, and control the second drive mechanism 21 to drive the detector 23 to move to a position below the bed platform 27.

The above describes implementation via the functional button(s) to rapid pre-imaging positioning of the X-ray head 13 and the detector 23 for the second imaging mode.

In examples where the X-ray imaging system 100 includes an image capturing device 33, the image capturing device 33 is configured to image a first image that comprises an optical image with two-dimensional spatial information and/or a depth image with three-dimensional spatial information.

In some embodiments, the processor 30 is further configured to: perform a third path planning based on the first image to control the first driving mechanism 11 to drive the X-ray head 13 to move a position over the bed platform 27. In some examples, the processor 30 is further configured to: perform a fourth path planning based on the first image to control the second driving mechanism 21 to drive the detector 23 to move to a position beneath the bed platform 27. In some examples, the processor 30 performs the third path planning based on the first image to control the first driving mechanism 11 to drive the X-ray head 13 to the third positioning point above the bed platform 27, and performs the fourth path planning based on the first image to control the second driving mechanism 21 to drive the detector 23 to the fourth positioning point below the bed platform 27, wherein the third positioning point and the fourth positioning point are matched such that the X-ray head 13 and the detector 23 satisfy a positional matching relationship required before imaging. In some examples, the positional matching relationship required before imaging includes the X-ray head 13 and the detector 23 meeting a predetermined distance requirement and/or a predetermined angular requirement. For example, the positional matching relationship may require the X-ray head 13 to be directly opposed to the detector 23, meaning an emission axis of the X-ray head 13 passes through a center of the detector 23 and is perpendicular to a plane where the detector 23 locates. It should be noted that the X-rays emitted from the X-ray head 13 generally form a conical X-ray beam, and the emission axis of the X-ray head 13 refers to the central axis of the conical X-ray beam.

In examples where the X-ray imaging system 100 includes the base station 34 and the tag 35, the processor 30 is further configured to: calculate relative position information between the base station 34 and the tag 35 based on signals transmitted and received by the base station 34, and control the first drive mechanism 11 to drive the X-ray head 13 to move based on this relative position information, ensuring that the X-ray head 13 and the detector 23 satisfy a positional matching relationship required before imaging. In some examples, the positional matching relationship required before imaging includes the X-ray head 13 and the detector 23 meeting a predetermined distance requirement and/or a predetermined angular requirement. For example, the positional matching relationship may refer to the X-ray head 13 to be directly opposed to the detector 23, meaning an emission axis of the X-ray head 13 passes through a center of the detector 23 and is perpendicular to a plane where the detector 23 locates. It should be noted that the X-rays emitted from the X-ray head 13 generally form a conical X-ray beam, and the emission axis of the X-ray head 13 refers to the central axis of the conical X-ray beam.

In this way, an operator first places the detector 23 below the bed platform 27, and the processor 30 is then further configured to: calculate relative position information between the base station 34 and the tag 35 based on signals transmitted and received by the base station 34, and control the first driving mechanism 11 to drive the X-ray head 13 to move based on the relative position information, such that the X-ray head 13 and the detector 23 satisfy the positional matching relationship required before imaging.

By means of the aforementioned configurations, rapid pre-imaging positioning of the X-ray head 13 and the detector 23 for the second imaging mode can be achieved prior to imaging.

When performing imaging of the anteroposterior view of the second body part to be stitched, the X-ray head 13 and the detector 23 are required to cooperatively execute multiple imaging of the anteroposterior view of the second body part to be stitched. The following describes the coordination mechanism between the X-ray head 13 and the detector 23.

In some embodiments, the processor 30 controls the first drive mechanism 15 to move the X-ray head 13 to multiple second travel points that are distributed above the bed platform 27 along the first direction; and when the X-ray head 13 reaches each of the at least two second travel points, the X-ray head 13 is controlled to emit X-rays to the second body part to be stitched.

The above describes some operational movements of the X-ray head 13 during imaging. The following details how the detector 23 coordinates therewith.

In some embodiments, the processor 30 controls the second driving mechanism 21 to drive the detector 23 to move to a position corresponding to each second travel point that the X-ray head 13 passes and where the X-rays are emitted, and receive X-rays emitted from the X-ray head 13 at that second travel point and penetrating the second body part to be stitched, thereby obtaining the aforementioned at least two anteroposterior X-ray images.

In some embodiments, the detector 23 remains stationary below the bed platform 27; for example, the processor 30 controls the detector 23 to stay stationary beneath the bed platform 27; and the processor 30 controls the detector 23 to receive X-rays emitted by the X-ray head 13 at each second travel point that the X-ray head 13 passes and where the X-rays are emitted and penetrating the second body part to be stitched, thereby obtaining the aforementioned at least two anteroposterior X-ray images.

In examples where the detector 23 remains stationary to cooperate with the X-ray head 13, the detector 23 is designed with a relatively large size. This configuration enables the detector 23 to capture X-rays emitted by the X-ray head 13 at respective second travel point and penetrating through the anteroposterior view of the object under examination.

To determine coordination between the X-ray head 13 and the detector 23, the processor 30 acquires the second body part to be stitched of the object under examination and determines an exposure parameter set of the second body part to be stitched. In some examples, the exposure parameter set includes: multiple second travel points that are distributed above the bed platform 27 along the first direction and where the X-ray head 13 is controlled to move, and exposure parameters for the X-ray head 13 at at least two second travel points. In some embodiments, the exposure parameters include an irradiation field area.

In examples where the X-ray imaging system 100 includes an image capturing device 33, the imaging device 33 is configured to capture a second image comprising an optical image with two-dimensional spatial information and/or a depth image with three-dimensional spatial information. The processor 30 determining the exposure parameter set of the second body part to be stitched includes: the processor 30 obtaining a current positional relationship between the second body part to be stitched and the detector 23 based on the second image, and determining the exposure parameter set of the second body part to be stitched based on this current positional relationship.

In examples where the X-ray imaging system 100 includes the base station 34 and the tag 35, the processor 30 determining the exposure parameter set of the second body part to be stitched includes: the processor 30 acquiring the current positional relationship between the second body part to be stitched and the detector 23 according to the relative position information, and determining the exposure parameter set of the second body part to be stitched based on this current positional relationship.

In some examples, the processor 30 determining the exposure parameter set of the second body part to be stitched includes: the processor 30 acquiring a predetermined positional relationship between the second body part to be stitched and the detector 23 when the object under examination is in a supine posture, and determining the exposure parameter set of the second body part to be stitched based on this predetermined positional relationship.

In some examples, a body part to be stitched (e.g., the second body part to be stitched) is preconfigured with its corresponding exposure parameter set; the object under examination is guided by an operator to carry out corresponding positioning, and the processor 30 controls the X-ray head 13 and the detector 23 to execute imaging of the body part to be stitched of the object under examination according to the preconfigured exposure parameter set corresponding to the current body part to be stitched.

In some embodiments, in response to the exposure instruction for the second body part to be stitched, the processor 30 controls the X-ray head 13 and the detector 23 to cooperate according to the exposure parameter set to image the second body part to be stitched of the object under examination in a supine posture, thereby obtaining at least two anteroposterior X-ray images of the second body part to be stitched.

For details of how the processor 30 controls cooperation between the X-ray head 13 and the detector 23 according to the exposure parameter set, reference may be made to the above descriptions. For example, the processor 30 controls the first drive mechanism 15 to move the X-ray head 13 to pass each of the at least two second travel points according to the exposure parameter set, and when the X-ray head 13 reaches said each of the at least two second travel points, the processor 30 controls the X-ray head 13 to emit X-rays to the second body part to be stitched of the object under examination in a supine posture on the bed platform 27 according to the exposure parameters for said each second travel point; and the processor 30 controls the detector 23 beneath the bed platform 27 to receive X-rays penetrating the second body part to be stitched, thereby obtaining at least two anteroposterior X-ray images of the second body part to be stitched.

After acquiring the at least two anteroposterior X-ray images, the processor 30 stitches these at least two anteroposterior X-ray images to obtain a radiograph of the second body part to be stitched. It should be understood that during image stitching, the processor may, after obtaining all anteroposterior X-ray images of the second body part to be stitched (i.e., after imaging exposure), stitch these anteroposterior X-ray images to obtain the radiograph of the second body part to be stitched. When performing image stitching, the processor may alternatively execute stitching during the imaging process, wherein image stitching can be performed immediately upon acquiring a single anteroposterior X-ray image, enabling concurrent operation of both image stitching and image acquisition procedures.

In some embodiments, the processor 30 is further configured to: acquire a region of interest (ROI) of the second body part to be stitched. The processor 30 stitching the at least two anteroposterior X-ray images to obtain the radiograph of the second body part to be stitched includes: determining a stitching region between adjacent anteroposterior X-ray images based on the ROI, ensuring that the ROI of the second body part to be stitched resides outside the stitching region.

In some embodiments, the processor 30 is further configured to: acquire the ROI of the second body part to be stitched, and determine the exposure parameter set based on the ROI, for example, determining second travel points associated with the ROI to ensure complete imaging of the ROI during imaging exposure while maintaining the ROI outside the stitching region. In other words, the processor 30 determines the exposure parameter set of the second body part to be stitched to ensure the ROI is fully captured during X-ray imaging and remains outside the stitching region.

The above describes implementations of imaging of anteroposterior view of the object under examination (or the second imaging mode).

In some embodiments, the X-ray imaging system 100 supports one or more imaging modes. For example, the X-ray imaging system 100 may include a first imaging mode for imaging the first body part to be stitched and/or a second imaging mode for imaging the second body part to be stitched. For another example, the X-ray imaging system 100 includes both the first imaging mode for imaging the first body part to be stitched and the second imaging mode for imaging the second body part to be stitched.

Thus, in some examples, the X-ray imaging system 100 includes: the first imaging mode for imaging the first body part to be stitched and the second imaging mode for imaging the second body part to be stitched; when the current imaging mode is the first imaging mode, the X-ray head 13 and the detector 23 are controlled to cooperatively image the first body part to be stitched of the object under examination in a supine posture to obtain at least two lateral X-ray images of the first body part to be stitched, wherein the radiograph of the first body part to be stitched is obtained by stitching the at least two lateral X-ray images by the processor 30; and when the current imaging mode is the second imaging mode, the processor 30 controls the X-ray head 13 and the detector 23to cooperatively image the second body part to be stitched of the object under examination in a supine posture to obtain at least two anteroposterior X-ray images of the second body part to be stitched, wherein the radiograph of the second body part to be stitched is obtained by stitching the at least two anteroposterior X-ray images by the processor 30. Further details of the first and second imaging modes are consistent with the descriptions provided above and are not reiterated here.

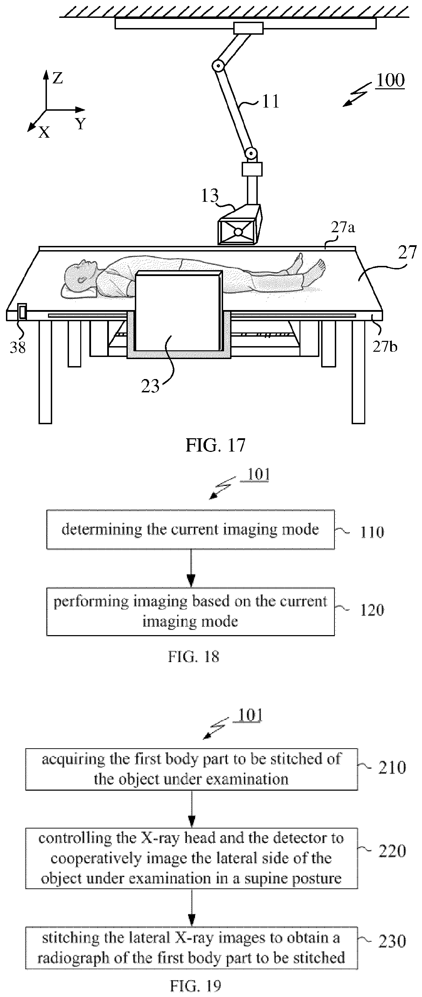

Please refer to FIG. 17, in some embodiments, the X-ray imaging system 100 further includes a mode switching interface 38 configured to be capable of being triggered by an operator to switch imaging modes. For example, when the current imaging mode is the first imaging mode, triggering the mode switching interface 38 by the operator switches the current imaging mode from the first imaging mode to the second imaging mode; and when the current imaging mode is the second imaging mode, triggering the mode switching interface 38 by the operator switches the current imaging mode from the second imaging mode to the first imaging mode.

The mode switching interface 38 may be a physical functional button, wherein the form of the physical functional button may include, but is not limited to, button-type, knob-type, or toggle lever-type configurations. The mode switching interface 38 may also be a virtual functional button, wherein the form of the virtual functional button may include an operational control element on a display interface, which can be presented as a menu or a simulated physical control element mimicking an actual key. The mode switching interface 38 may be disposed on the bed platform 27, the second support assembly 2, or the X-ray head 13.

The mode switching interface 38 not only enables rapid configuration of imaging modes but also provides a convenient means to position the X-ray head 13 and/or the detector 23 to locations corresponding to the current imaging mode prior to imaging.

For example, in response to a triggering operation on the mode switching interface 38, the processor 30 determines the current imaging mode. When the current imaging mode is switched to the first imaging mode, the processor 30 controls the first drive mechanism 11 to move the X-ray head 13 to the first side 27a of the bed platform 27, and/or controls the second drive mechanism 21 to move the detector 23 to the second side 27b of the bed platform 27. For instance, the processor 30 directs the X-ray head 13 to a first positioning point at the first side 27a of the bed platform 27 and directs the detector 23 to a second positioning point at the second side 27b of the bed platform 27, where the first positioning point matches the second positioning point so that the X-ray head 13 and the detector 23 satisfy positional matching relationship required before imaging. When the current imaging mode is switched to the second imaging mode, the processor 30 controls the first drive mechanism 11 to move the X-ray head 13 to a position over the bed platform 27, and/or controls the second drive mechanism 21 to move the detector 23 to a position below the bed platform 27. For example, the processor 30 directs the X-ray head 13 to a third positioning point above the bed platform 27 and directs the detector 23 to a fourth positioning point below the bed platform 27, where the third positioning point matches the fourth positioning point, so that the X-ray head 13 and the detector 23 satisfy a positional matching relationship required before imaging. The positional matching relationship required before imaging may be referenced as previously described and will not be reiterated here.

In some examples where the X-ray imaging system 100 includes the image capturing device 33, the image capturing device 33 is configured to capture a first image comprising an optical image with two-dimensional spatial information and/or a depth image with three-dimensional spatial information.

In some examples, when the current imaging mode is switched to the first imaging mode, the processor 30 performs the first path planning based on the first image to control the first drive mechanism 11 to move the X-ray head 13 to the first side 27a of the bed platform 27, such as the first positioning point at the first side 27a.

In some examples, when the current imaging mode is switched to the first imaging mode, the processor 30 performs the second path planning based on the first image to control the second drive mechanism 21 to move the detector 23 to the second side 27b of the bed platform 27, such as the second positioning point at the second side 27b.

In some examples, the first positioning point matches the second positioning point so that the X-ray head 13 and the detector 23 satisfy the positional matching relationship required before imaging.

In some examples, when the current imaging mode is switched to the second imaging mode, the processor 30 performs the third path planning based on the first image to control the first drive mechanism 11 to move the X-ray head 13 to a position over the bed platform 27, such as the third positioning point above the bed platform 27.

In some examples, when the current imaging mode is switched to the second imaging mode, the processor 30 performs the fourth path planning based on the first image to control the second drive mechanism 21 to move the detector 23 to a position below the bed platform 27, such as the fourth positioning point below the bed platform 27.

In some examples, the third positioning point matches the fourth positioning point so that the X-ray head 13 and the detector 23 satisfy the positional matching relationship required before imaging.

The positional matching relationship required before imaging may be referenced as previously described and will not be reiterated here.

The foregoing provides illustrative descriptions of the X-ray imaging system 100.

An imaging method 101 for an X-ray imaging system 100 is also disclosed in some embodiments, wherein said X-ray imaging system 100 involved in the imaging method 101 may be the X-ray imaging system 100 described in some embodiments herein.

Referring to FIG. 18, the imaging method 101 in some embodiments includes the following steps:
Step 110: determining the current imaging mode.

In some examples, the imaging mode includes the first imaging mode for the and the second imaging mode for the second body part to be stitched.

In some embodiments, in step 110, the current imaging mode is determined in response to an operation of the mode switching interface 38. For example, when the current imaging mode is the first imaging mode, triggering the mode switching interface 38 by an operator may switch the current imaging mode from the first imaging mode to the second imaging mode; and when the current imaging mode is the second imaging mode, triggering the mode switching interface 38 by the operator may switch the current imaging mode from the second imaging mode to the first imaging mode.

The mode switching interface 38 not only enables rapid configuration of the imaging mode but also provides a convenient way to position the X-ray head 13 and/or the detector 23 to a position corresponding to the current imaging mode prior to imaging.

In response to a triggering operation of the mode switching interface 38, in step 110, the current imaging mode is determined, wherein when the current imaging mode is switched to the first imaging mode, step 110 further involves controlling the X-ray head 13 to move to the first side 27a of the bed platform 27 and/or controlling the detector 23 to move to the second side 27b of the bed platform 27. For example, step 110 involves controlling the X-ray head 13 to move to the first positioning point at the first side 27a of the bed platform 27 and controlling the detector 23 to move to the second positioning point at the second side 27b of the bed platform 27. The first positioning point and the second positioning point are matched to ensure that the X-ray head 13 and the detector 23 satisfy a positional matching relationship required before imaging. When the current imaging mode is switched to the second imaging mode, step 110 involves controlling the X-ray head 13 to move to a position over the bed platform 27 and/or controlling the detector 23 to move to a position below the bed platform 27. For example, step 110 involves controlling the X-ray head 13 to move to a third positioning point above the bed platform 27 and controlling the detector 23 to move to a fourth positioning point below the bed platform 27. The third positioning point and the fourth positioning point are matched to ensure that the X-ray head 13 and the detector 23 satisfy the positional matching relationship required before imaging. The positional matching relationship required before imaging may refer to the descriptions above, which will not be reiterated here.

Step 120: performing imaging based on the current imaging mode.

In some examples, when the current imaging mode is the first imaging mode, step 120 involves controlling the X-ray head 13 and the detector 23 to cooperatively image the first body part to be stitched of the object under examination in a supine posture, obtaining at least two lateral X-ray images of the first body part to be stitched. A radiograph of the first body part to be stitched is obtained by stitching the at least two lateral X-ray images.

In some embodiments, step 120 involves controlling the X-ray head 13 and the detector 23 to cooperatively image the first body part to be stitched of the object under examination in a supine posture, obtaining at least two lateral X-ray images of the first body part to be stitched, which may be implemented by: controlling the X-ray head 13 to move to multiple first travel points distributed along the first direction at the first side 27a of the bed platform 27; and when the X-ray head 13 moves to each of at least two first travel points, controlling the X-ray head 13 to emit X-rays to the first body part to be stitched.

In some embodiments, step 120 involves controlling the X-ray head 13 and the detector 23 to cooperatively image the first body part to be stitched of the object under examination in a supine posture to obtain at least two lateral X-ray images of the first body part to be stitched, which may be implemented by: the X-ray head 13 being manually moved by an operator-applied force to move to a plurality of first travel points distributed along the first direction at the first side 27a of the bed platform 27; and when the X-ray head 13 is moved to each of at least two first travel points, controlling the X-ray head 13 to emit X-rays to the first body part to be stitched.

In some embodiments, step 120 involves controlling the X-ray head 13 and the detector to cooperatively image the first body part to be stitched of the object under examination in a supine posture to obtain at least two lateral X-ray images of the first body part to be stitched, which may further include: controlling the detector 23 to move to a position corresponding to each first travel point that the X-ray head 13 passes and where the X-rays are emitted and receive X-rays emitted by the X-ray head 13 at that first travel point and penetrating the first body part to be stitched, thereby obtaining at least two lateral X-ray images.

In some embodiments, step 120 involves controlling the X-ray head 13 and the detector to cooperatively image the first body part to be stitched of the object under examination in a supine posture to obtain at least two lateral X-ray images of the first body part to be stitched, which may further include: controlling the detector 23 to remain stationary at the second side 27b of the bed platform 27, and controlling the detector 13 to receive X-rays emitted by the X-ray head 13 at each first travel point where the X-rays are emitted and penetrating the first body part to be stitched, thereby obtaining the at least two lateral X-ray images.

In some embodiments, step 120 involves controlling the X-ray head 13 and the detector to cooperatively image the first body part to be stitched of the object under examination in a supine posture to obtain at least two lateral X-ray images of the first body part to be stitched, which may further include: the X-ray head 13 being remained stationary at the first side 27a of the bed platform 27, controlling the window 42 of the beam-limiting window assembly 40 to sequentially move to a plurality of first positions distributed along the first direction, and controlling the detector 23 to sequentially move to a plurality of second positions corresponding to the plurality of first positions and receive X-rays penetrating the first body part to be stitched via the window 42 at each second position, thereby obtaining the at least two lateral X-ray images.

When the current imaging mode is the second imaging mode, step 120 involves controlling the X-ray head 13 and the detector 23 to cooperatively image the second body part to be stitched of the object under examination in a supine posture to obtain at least two anteroposterior X-ray images of the second body part to be stitched, wherein a radiograph of the second body part to be stitched is generated by stitching the at least two anteroposterior X-ray images.

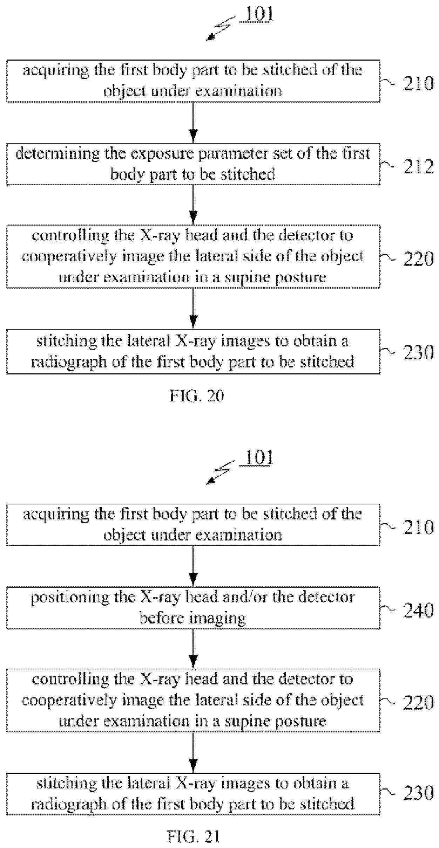

As shown in FIG. 19 or FIG. 20, the imaging method 101 in some embodiments includes the following steps:
Step 210: acquiring the first body part to be stitched of the object under examination.
Step 212: determining the exposure parameter set of the first body part to be stitched.

In some examples, the exposure parameter set includes: multiple first travel points that are distributed along the first direction at the first side 27a of the bed platform 27 and where the X-ray head 13 is controlled to move, and exposure parameters for the X-ray head 13 at at least two first travel points. In some embodiments, the exposure parameters include an irradiation field area.

In some embodiments, in step 212, a current positional relationship between the first body part to be stitched and a detector 23 is acquired based on the second image, and the exposure parameter set of the first body part to be stitched is determined based on the current positional relationship, where the second image includes an optical image with two-dimensional spatial information and/or a depth image with three-dimensional spatial information.

In some embodiments, in step 212, the relative position information between the base station 34 and the tags 35 is calculated based on signals transmitted and received by the base station 34, the current positional relationship between the first body part to be stitched and the detector 23 is acquired based on the relative position information, and the exposure parameter set of the first body part to be stitched is determined based on the current positional relationship.

In some embodiments, in step 212, a predetermined positional relationship between the first body part to be stitched and the detector 23 is acquired when the object under examination is in a supine posture, and the exposure parameter set of the first body part to be stitched is determined based on the predetermined positional relationship.

Step 220: controlling the X-ray head 13 and the detector 23 to cooperatively image the lateral view of the object under examination in a supine posture. In some embodiments, step 220 includes: in response to an exposure instruction for the first body part to be stitched, controlling the X-ray head 13 and the detector 23 to cooperatively image the first body part to be stitched of the object under examination in a supine posture, thereby obtaining at least two lateral X-ray images of the first body part to be stitched.

For example, step 220 includes: in response to the exposure instruction for the first body part to be stitched, controlling the X-ray head 13 and the detector 23 according to the exposure parameter set of the first body part to be stitched to cooperatively image the first body part to be stitched of the object under examination in a supine posture, thereby obtaining at least two lateral X-ray images of the first body part to be stitched.

In some embodiments, controlling the X-ray head 13 and the detector 23 to cooperatively image the first body part to be stitched of the object under examination in a supine posture to obtain at least two lateral X-ray images of the first body part to be stitched in step 220 may comprise: controlling the X-ray head 13 to move to multiple first travel points distributed at the first side 27a of the bed platform 27 along the first direction; and when the X-ray head 13 moves to each of at least two first travel points, controlling the X-ray head 13 to emit X-rays to the first body part to be stitched.

In some embodiments, controlling the X-ray head 13 and the detector 23 to cooperatively image the first body part to be stitched of the object under examination in a supine posture to obtain at least two lateral X-ray images of the first body part to be stitched in step 220 may comprise: the X-ray head 13 being manually moved by an operator through applied force to multiple first travel points distributed at the first side 27a of the bed platform 27 along the first direction; and when the X-ray head 13 moves to each of at least two first travel points, controlling the X-ray head 13 to emit X-rays to the first body part to be stitched.

In some embodiments, controlling the X-ray head 13 and the detector 23 to cooperatively image the first body part to be stitched of the object under examination in a supine posture to obtain at least two lateral X-ray images of the first body part to be stitched in step 220 may also comprise: controlling the detector 23 to move to a position corresponding to each first travel point that the X-ray head 13 passes and where the X-rays are emitted, and receive X-rays emitted by the X-ray head 13 at that first travel point and penetrating the first body part to be stitched, thereby obtaining the at least two lateral X-ray images.

In some embodiments, controlling the X-ray head 13 and the detector 23 to cooperatively image the first body part to be stitched of the object under examination in a supine posture to obtain at least two lateral X-ray images of the first body part to be stitched in step 220 may also comprise: controlling the detector 23 to remain stationary at the second side 27b of the bed platform 27, and controlling the detector 23 to receive X-rays emitted from the X-ray head 13 at each first travel point and penetrating the first body part to be stitched, thereby obtaining the at least two lateral X-ray images.

In some embodiments, controlling the X-ray head 13 and the detector 23 to cooperatively image the first body part to be stitched of the object under examination in a supine posture to obtain at least two lateral X-ray images of the first body part to be stitched in step 220 may also comprise: the X-ray head 13 being remained stationary at the first side 27a of the bed platform 27, the window 42 of the beam-limiting window assembly 40 being controlled to sequentially move to multiple first positions distributed along the first direction, and the detector 23 being controlled to sequentially move to multiple second positions corresponding to the multiple first positions and receive X-rays penetrating the first body part to be stitched via the window 42 at each second position, thereby obtaining at least two lateral X-ray images.

Step 230: stitching the at least two lateral X-ray images to obtain a radiograph of the first body part to be stitched.

In some embodiments, step 230 further involves acquiring a region of interest (ROI) of the first body part to be stitched; and during stitching of the at least two lateral X-ray images, in step 230, a stitching region between adjacent lateral X-ray images is determined based on the ROI, ensuring that the ROI of the first body part to be stitched locates outside the stitching region.

As shown in FIG. 21, the imaging method 101 in some embodiments includes step 240: positioning the X-ray head 13 and/or the detector 23 before imaging, as described below.

In some embodiments, before imaging: in step 240, in response to triggering of the first positioning button 31, the X-ray head 13 is controlled to move to the first side 27a of the bed platform 27; and/or in response to triggering of the second positioning button 32, the detector 23 is controlled to move to the second side 27b of the bed platform 27. Descriptions of the first positioning button 31 and second positioning button 32 are provided above and will not be repeated here.

The first positioning button 31 and the second positioning button 32 respectively trigger the X-ray head 13 to move to the first side 27a of the bed platform 27 and the detector 23 to move to the second side 27b of the bed platform 27. To further optimize operational efficiency and time savings, the first positioning button 31 may be triggered to drive the X-ray head 13 to a first positioning point at the first side 27a of the bed platform 27, while the second positioning button 32 may be triggered to drive the detector 23 to a second positioning point at the second side 27b of the bed platform 27, wherein the first positioning point and the second positioning point are mutually aligned such that the X-ray head 13 and the detector 23 satisfy a positional matching relationship required before imaging. In some example, the positional matching relationship required before imaging include the X-ray head 13 and the detector 23 meeting a predetermined distance requirement and/or a predetermined angular requirement. For example, the positional matching relationship may require the X-ray head 13 to be directly opposed to the detector 23, wherein an emission axis of the X-ray head 13 passes through the center of the detector 23 and is perpendicular to a plane where the detector 23 locates. It is noted that the X-ray emitted by the X-ray head 13 is generally a conical X-ray beam, and the emission axis of the X-ray head 13 refers to the central axis of the conical X-ray beam.

In some embodiments, before imaging: in step 240, the first image is acquired, wherein the first imaging comprises an optical image with two-dimensional spatial information and/or a depth image with three-dimensional spatial information; step 240 involves performing the first path planning based on the first image to control movement of the X-ray head 13 to the first side 27a of the bed platform 27, and/or performing the second path planning based on the first image to control movement of the detector 23 to the second side 27b of the bed platform 27. In some examples, step 240 involves performing the first path planning to move the X-ray head 13 to a first positioning point at the first side 27a of the bed platform 27, and performing the second path planning to move the detector 23 to a second positioning point at the second side 27b of the bed platform 27, where the first positioning point matches the second positioning point, such that the X-ray head 13 and the detector 23 satisfy a positional matching relationship required before imaging. In some examples, the positional matching relationship required before imaging includes the X-ray head 13 and the detector satisfy a predetermined distance requirement and/or a predetermined angular requirement. For example, the positional matching relationship may require the X-ray head 13 to be directly opposed to the detector 23, wherein an emission axis of the X-ray head 13 passes through the center of the detector 23 and is perpendicular to a plane where the detector 23 locates. It is noted that the X-ray emitted by the X-ray head 13 is generally a conical X-ray beam, and the emission axis of the X-ray head 13 refers to the central axis of the conical X-ray beam.

This disclosure describes, with reference to various exemplary embodiments. However, Those skilled in the art will recognize that modifications and changes may be made to the exemplary embodiments without departing from the scope of this document. For instance, various operational steps, as well as the components used to execute these steps, may be implemented in different manners, taking into account specific applications or various cost functions related to system operation (e.g., one or more steps may be omitted, modified, or combined with other steps).

In the above-mentioned embodiments, it can be implemented fully or partially through software, hardware, firmware, or any combination thereof. Additionally, as understood by those skilled in the art, the principles presented herein may be embodied in a computer program product reflected on a computer-readable storage medium, wherein the readable storage medium is pre-installed with computer-readable program code. Any tangible, non-transitory computer-readable storage medium may be utilized, including magnetic storage devices (hard disks, floppy disks, etc.), optical storage devices (CD-ROMs, DVDs, Blu-ray discs, etc.), flash memory, and/or the like. These computer program instructions can be loaded onto general-purpose computers, special-purpose computers, or other programmable data processing devices to form a machine, enabling the instructions executed on these computers or other programmable data processing devices to generate devices that perform specified functions. These computer program instructions can also be stored in a computer-readable memory, which can instruct a computer or other programmable data processing device to operate in a specific manner, such that the instructions stored in the computer-readable memory can form an article of manufacture, including an implementation device that realizes the specified functions. Furthermore, computer program instructions can be loaded onto a computer or other programmable data processing device, thereby executing a series of operational steps on the computer or other programmable device to produce a computer-implemented process. This allows the instructions executed on the computer or other programmable device to provide steps for realizing specified functions.

While the principles disclosed herein have been illustrated through various embodiments, it should be understood that structural configurations, material selections, and component proportions particularly suited to specific operational environments may be modified without departing from the scope and spirit of the disclosure. Such modifications, along with other adaptations or adjustments, shall be encompassed within the scope of the present disclosure.

The foregoing detailed description has been described with reference to various embodiments. However, those skilled in the art will recognize that modifications and variations may be made without departing from the scope of the disclosure. Accordingly, the description of the disclosure shall be interpreted in an illustrative rather than restrictive sense, and all such modifications are intended to be included within its scope. Similarly, discussions of advantages, alternative solutions to problems, and operational benefits associated with the embodiments are provided above. Nevertheless, benefits, advantages, solutions to problems, and any elements that may produce such effects or render them more explicit shall not be construed as critical, required, or essential. Furthermore, the term 'coupled' and its derivatives encompass physical connections (e.g., mechanical joints), electrical connections (e.g., circuit interconnects), magnetic linkages (e.g., inductive coupling), optical interfaces (e.g., fiber-optic alignment), communication channels (e.g., wireless protocols), functional integrations (e.g., software APIs), and any other form of association that achieves operational interaction.

Those skilled in the art will recognize that numerous modifications to the details of the above-described embodiments may be made without departing from the fundamental principles of the disclosed subject matter. Accordingly, the scope of the present disclosure shall be determined solely by the claims and their legal equivalents.

## Claims

1. An X-ray imaging system, comprising an X-ray head device, an X-ray receiving device and a processor; wherein
the X-ray head device comprises a first support assembly, a first drive mechanism, and an X-ray head; wherein the X-ray head is configured to emit X-rays to an object under examination and is movably disposed on the first support assembly, and the first drive mechanism is configured to drive the X-ray head to move;
the X-ray receiving device comprises a bed platform, a second support assembly, and a detector; wherein the bed platform is configured to support the object under examination who is lying in a supine posture along a first direction of the bed platform, the bed platform has a first side and a second side that are distributed along the first direction, the first side and the second side are opposite sides of the bed platform; the second support assembly is configured to support the detector; the detector is configured to receive X-rays penetrating the object under examination; the X-ray head is capable of being positioned at the first side of the bed platform via the first support assembly, and the detector is capable of being positioned at the second side of the bed platform via the second support assembly, such that the X-ray head and the detector are faced toward a lateral view of the object under examination in a supine posture so as to obtain a lateral X-ray image of the object under examination; and
the processor is configured to:
acquire a first body part to be stitched of the object under examination;
determine an exposure parameter set of the first body part to be stitched, wherein the exposure parameter set comprises: a plurality of first travel points, to which the X-ray head is controlled to move, that are distributed at the first side of the bed platform along the first direction, and exposure parameters for the X-ray head at at least two of the first travel points;
in response to an exposure instruction for the first body part to be stitched, control, based on the exposure parameter set, the first drive mechanism to drive the X-ray head to pass each of the at least two of the first travel points; and when the X-ray head is moved to said each first travel point, control, based on the exposure parameters for said each first travel point, the X-ray head to emit X-rays to the first body part to be stitched of the object under examination in a supine posture;
control the detector at the second side of the bed platform to receive X-rays penetrating the first body part to be stitched, thereby obtaining at least two lateral X-ray images of the first body part to be stitched; and
stitch the at least two lateral X-ray images to obtain a radiograph of the first body part to be stitched.

2. The X-ray imaging system according to claim 1, wherein
the X-ray receiving device further comprises a second drive mechanism configured to drive the detector to move; and
the processor being configured to control the detector at the second side of the bed platform to receive X-rays penetrating the first body part to be stitched, thereby obtaining at least two lateral X-ray images of the first body part to be stitched, comprises:
the processor being configured to control the second drive mechanism to drive the detector to move to a position corresponding to said each first travel point, where the X-ray head passes and emits the X-rays, and to receive the X-rays emitted by the X-ray head at said each first travel point and penetrating the first body part to be stitched, thereby obtaining the at least two lateral X-ray images;
or, the processor being configured to control the detector to remain stationary at the second side of the bed platform, and control the detector to receive the X-rays emitted by the X-ray head at each of the at least two of the first travel points, to which the X-ray head is moved, and penetrating the first body part to be stitched, thereby obtaining the at least two lateral X-ray images.

3. The X-ray imaging system according to claim 1, further comprising at least one of the following:
a first positioning button, capable of being triggered by an operator to cause the processor to control the first drive mechanism to drive the X-ray head to move to the first side of the bed platform; and
an image capturing device, configured to capture a first image that comprises at least one of: an optical image with two-dimensional spatial information, and a depth image with three-dimensional spatial information; wherein the processor is further configured to perform a first path planning based on the first image, so as to control the first drive mechanism to drive the X-ray head to move to the first side of the bed platform.

4. The X-ray imaging system according to claim 3, wherein:
the X-ray receiving device further comprises a second drive mechanism configured to drive the detector to move;
the X-ray imaging system further comprises a second positioning button capable of being triggered by an operator to cause the processor to control the second drive mechanism to drive the detector to move to the second side of the bed platform, wherein the second positioning button and the first positioning button are a same functional button or different functional buttons;
and/or, the processor is further configured to perform a second path planning based on the first image, so as to control the second drive mechanism to drive the detector to move to the second side of the bed platform.

5. The X-ray imaging system according to claim 1, wherein
the X-ray imaging system further comprises an image capturing device configured to capture a second image that comprises at least one of: an optical image with two-dimensional spatial information, and a depth image with three-dimensional spatial information; wherein the processor being configured to determine the exposure parameter set of the first body part to be stitched, comprises: the processor being configured to acquire a current positional relationship between the first body part to be stitched and the detector based on the second image, and determine the exposure parameter set of the first body part to be stitched according to the current positional relationship;
or, the X-ray imaging system further comprises at least a base station disposed on the X-ray head and at least a tag disposed on the detector, wherein the base station is configured to transmit signals to the tag and receive signals from the tag; the processor is further configured to calculate relative position information between the base station and the tag based on the signals transmitted and received by the base station; and the processor being configured to determine the exposure parameter set of the first body part to be stitched, comprises: the processor being configured to acquire a current positional relationship between the first body part to be stitched and the detector based on the relative position information, and determine the exposure parameter set of the first body part to be stitched according to the current positional relationship;
or, the processor being configured to determine the exposure parameter set of the first body part to be stitched, comprises: the processor being configured to acquire a predetermined positional relationship between the first body part to be stitched and the detector when the object under examination is in a supine posture, and determine the exposure parameter set of the first body part to be stitched according to the predetermined positional relationship.

6. The X-ray imaging system according to any one of claims 1 to 5, wherein:
the processor is further configured to acquire a region of interest of the first body part to be stitched; and
the processor being configured to stitch the at least two lateral X-ray images to obtain the radiograph of the first body part to be stitched, comprises: the processor being configured to determine a stitching region between adjacent lateral X-ray images based on the region of interest, such that the region of interest of the first body part to be stitched is outside the stitching region.

7. The X-ray imaging system according to claim 1, wherein the second support assembly comprises a mounting bracket configured to detachably mount the detector, wherein the mounting bracket is disposed on the second side of the bed platform and movable along the first direction, so that the detector is capable of being positioned at the second side of the bed platform and driven to move with the mounting bracket.

8. The X-ray imaging system according to claim 1, further comprising: at least a base station disposed on the X-ray head, and at least a tag disposed on the detector; wherein the base station is configured to transmit signals to the tag and receive signals from the tag; and
the processor is further configured to:
calculate relative position information between the base station and the tag based on the signals transmitted and received by the base station; and
control the first drive mechanism to drive the X-ray head to move according to the relative position information, so that the X-ray head and the detector satisfy a positional matching relationship required before imaging.

9. The X-ray imaging system according to claim 8, wherein the positional matching relationship required before imaging comprises the X-ray head and the detector satisfying at least one of: a predetermined distance requirement, and a predetermined angular requirement.

10. The X-ray imaging system according to claim 1, wherein the X-ray head is further capable of being positioned above the bed platform via the first support assembly, and the detector is further capable of being positioned below the bed platform via the second support assembly, such that the X-ray head and the detector are faced toward an anteroposterior view of the object under examination in a supine posture so as to obtain an anteroposterior X-ray image of the object under examination.

11. An X-ray imaging system, comprising an X-ray head device, an X-ray receiving device, and a processor; wherein
the X-ray head device comprises a first support assembly configured to emit X-rays to an object under examination, and an X-ray head movably disposed on the first support assembly;
the X-ray receiving device comprises a bed platform, a second support assembly, and a detector; wherein the bed platform is configured to support the object under examination who is lying in a supine posture along a first direction of the bed platform, the bed platform has a first side and a second side that are distributed along the first direction, the first side and the second side are opposite sides of the bed platform; the second support assembly is configured to support the detector; the detector is configured to receive X-rays penetrating the object under examination; wherein the X-ray head is capable of being positioned at the first side of the bed platform via the first support assembly, and the detector is capable of being positioned at the second side of the bed platform via the second support assembly, such that the X-ray head and the detector are faced toward a lateral view of the object under examination in a supine posture so as to obtain a lateral X-ray image of the object under examination;
the processor is configured to:
acquire a first body part to be stitched of the object under examination;
in response to an exposure instruction for the first body part to be stitched, control the X-ray head and the detector to cooperatively image the first body part to be stitched of the object under examination in a supine posture, thereby obtaining at least two lateral X-ray images of the first body part to be stitched; and
stitch the at least two lateral X-ray images to obtain a radiograph of the first body part to be stitched.

12. The X-ray imaging system according to claim 11, wherein
the processor being configured to control the X-ray head and the detector to cooperatively image the first body part to be stitched of the object under examination in a supine posture, thereby obtaining at least two lateral X-ray images of the first body part to be stitched, comprises:
the processor being configured to control a first drive mechanism to drive the X-ray head to move to a plurality of first travel points that are distributed at the first side of the bed platform along the first direction; and when the X-ray head is moved to each of at least two of the first travel points, controlling the X-ray head to emit X-rays to the first body part to be stitched, wherein the X-ray head device further comprises the first drive mechanism;
or, the X-ray head is manually moved by an operator-applied force to a plurality of first travel points that are distributed at the first side of the bed platform along the first direction; and when the X-ray head is moved to each of at least two of the first travel points, the processor is further configured to control the X-ray head to emit X-rays to the first body part to be stitched.

13. The X-ray imaging system according to claim 12, wherein
the processor being configured to control the X-ray head and the detector to cooperatively image the first body part to be stitched of the object under examination in a supine posture, thereby obtaining at least two lateral X-ray images of the first body part to be stitched, further comprises:
the processor being configured to control a second drive mechanism to drive the detector to move to a position corresponding to each of the at least two of the first travel points, where the X-ray head passes and emits the X-rays, and to receive the X-rays emitted by the X-ray head at said each first travel point and penetrating the first body part to be stitched, thereby obtaining the at least two lateral X-ray images, wherein the X-ray receiving device further comprises the second drive mechanism;
or, the processor being configured to control the detector to remain stationary at the second side of the bed platform, and control the detector to receive the X-rays emitted by the X-ray head at said each first travel point where the X-ray head passes and penetrating the first body part to be stitched, thereby obtaining the at least two lateral X-ray images.

14. The X-ray imaging system according to claim 11, further comprising: a beam-limiting window assembly disposed between the first body part to be stitched and the X-ray head, wherein the beam-limiting window assembly comprises a shielding portion for blocking X-rays and a window defined by the shielding portion for allowing passage of X-rays; and
wherein the processor being configured to control the X-ray head and the detector to cooperatively image the first body part to be stitched of the object under examination in a supine posture, thereby obtaining at least two lateral X-ray images of the first body part to be stitched, comprises: while the X-ray head is remained stationary at the first side of the bed platform,
the processor being configured to control the window of the beam-limiting window assembly to sequentially move to multiple first positions distributed along the first direction, and control the detector to sequentially move to multiple second positions corresponding to the multiple first positions and receive the X-rays penetrating the first body part to be stitched via the window at each of the second positions, thereby obtaining the at least two lateral X-ray images.
